# EUROPÄISCHE PATENTSCHRIFT

(11) **EP 1 474 407 B1**
(45) Veröffentlichungstag und Bekanntmachung des Hinweises auf die Patenterteilung: **04.08.2010**
(21) Anmeldenummer: 03737263.8
(22) Anmeldetag: 22.01.2003
(51) Int. Cl.: C07D 277/56, A01N 43/78

(54) **DIFLUORMETHYL THIAZOLYL CARBOXANILIDE**
DIFLUOROMETHYL THIAZOLYL CARBOXANILIDES
DIFLUOROMETHYL THIAZOLYL CARBOXANILIDES

(30) Priorität: 04.02.2002 DE 10204391
(43) Veröffentlichungstag der Anmeldung: 10.11.2004
(73) Patentinhaber: Bayer CropScience AG, 40789 Monheim (DE)
(72) Erfinder: ELBE, Hans-Ludwig, 42329 Wuppertal (DE); RIECK, Heiko, 69110 Ste. Foy-lès-Lyon (FR); DUNKEL, Ralf, 40789 Monheim (DE); WACHENDORFF-NEUMANN, Ulrike, 56566 Neuwied (DE); KUCK, Karl-Heinz, 40764 Langenfeld (DE); MAULER-MACHNIK, Astrid, 42799 Leichlingen (DE); KUGLER, Martin, 42799 Leichlingen (DE); JAETSCH, Thomas, 50668 Köln (DE); WACHTLER, Peter, 47800 Krefeld (DE)
(86) Internationale Anmeldenummer: PCT/EP2003/000589
(87) Internationale Veröffentlichungsnummer: WO 2003/066610

(56) Entgegenhaltungen:
- EP-A- 0 545 099
- WO-A-97/08148
- PATENT ABSTRACTS OF JAPAN vol. 2002, no. 02, 2. April 2002 (2002-04-02) & JP 2001 302605 A (SUMITOMO CHEM CO LTD), 31. Oktober 2001 (2001-10-31)

## Beschreibung

Die vorliegende Erfindung betrifft neue Difluormethylthiazolylcarboxanilide, mehrere Verfahren zu deren Herstellung und deren Verwendung zur Bekämpfung von schädlichen Mikroorganismen im Pflanzenschutz und Materialschutz.

Es ist bereits bekannt geworden, dass zahlreiche Carboxanilide fungizide Eigenschaften besitzen (vergleiche z.B. EP-A 0 545 099). Die Wirksamkeit der dort beschriebenen Stoffe ist gut, lässt aber bei niedrigen Aufwandmengen in manchen Fällen zu wünschen übrig.

Es wurden nun neue Difluormethylthiazolylcarboxanilide der Formel (I) 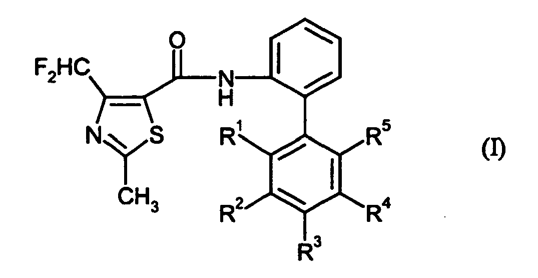 in welcher
- R¹, R², R³, R⁴ und R⁵: unabhängig voneinander für Wasserstoff, Halogen, Cyano, Nitro, C₁-C₆-Alkyl, C₂-C₆-Alkenyl, C₁-C₄-Alkoxy, C₁-C₄-Alkylthio, C₁-C₄- Alkylsulfonyl, C₃-C₆-Cycloalkyl, oder für C₁-C₄-Halogenalkyl, C₁-C₄-Halo- genalkoxy, C₁-C₄-Halogenalkylthio oder C₁-C₄-Halogenalkylsulfonyl mit jeweils 1 bis 5 Halogenatomen stehen, mit der Maßgabe, dass R¹, R², R³, R⁴ und R⁵ nicht gleichzeitig für Wasserstoff stehen,
- R¹ und R² oder R² und R³: außerdem gemeinsam für gegebenenfalls durch Halogen oder C₁-C₆-Alkyl substituiertes Alkenylen stehen.

Weiterhin wurde gefunden, dass man Difluormethylthiazolylcarboxanilide der Formel (I) erhält, indem man
a) Difluormethylthiazolylcarbonsäurehalogenide der Formel (II) 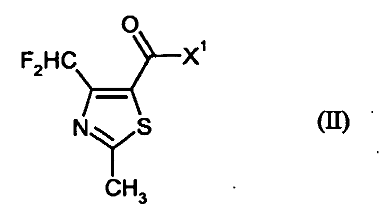 in welcher
   - X¹: für Halogen steht,
   mit Anilinderivaten der Formel (III) 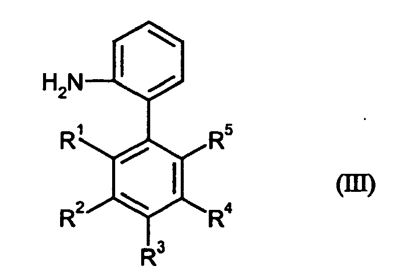 in welcher
   R¹, R², R³, R⁴ und R⁵ die oben angegebenen Bedeutungen haben,
   gegebenenfalls in Gegenwart eines Säurebindemittels und gegebenenfalls in Gegenwart eines Verdünnungsmittels umsetzt, oder
b) Difluormethylthiazolylcarboxhalogenanilide der Formel (IV) 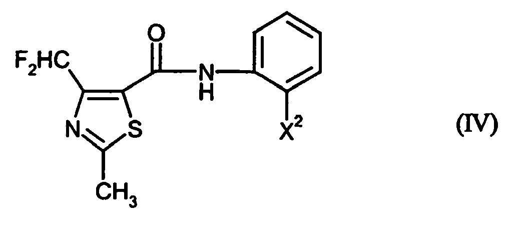 in welcher
   - X²: für Brom oder Iod steht,
   mit Boronsäurederivaten der Formel (V) 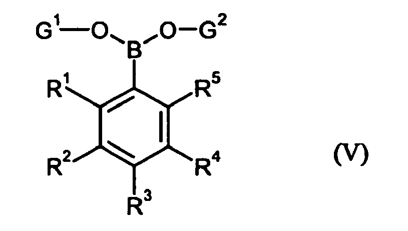 in welcher
   R¹, R², R³, R⁴ und R⁵ die oben angegebenen Bedeutungen haben,
   G¹ und G² jeweils für Wasserstoff oder zusammen für Tetramethylethylen stehen,
   in Gegenwart eines Katalysators, gegebenenfalls in Gegenwart eines Säurebindemittels und gegebenenfalls in Gegenwart eines Verdünnungsmittels umsetzt, oder
c) Difluormethylthiazolylcarboxanilid-Boronsäure-Derivate der Formel (VI) 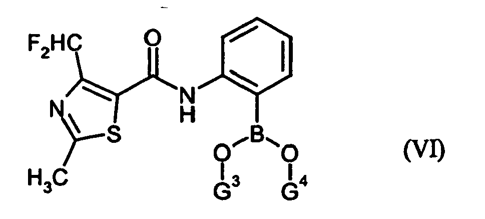 in welcher
   G³ und G⁴ jeweils für Wasserstoff oder zusammen für Tetramethylethylen stehen,
   mit Halogenbenzolderivaten der Formel (VII) 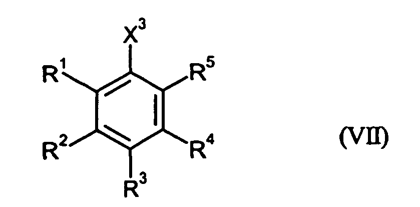 in welcher
   - R¹, R², R³, R⁴ und R⁵: die oben angegebenen Bedeutungen haben,
   - X³: für Brom, Iod oder Trifluormethylsulfonyloxy steht,
in Gegenwart eines Katalysators, gegebenenfalls in Gegenwart eines Säurebindemittels und gegebenenfalls in Gegenwart eines Verdünnungsmittels umsetzt.

Schließlich wurde gefunden, dass die neuen Difluormethylthiazolylcarboxanilide der Formel (I) sehr gute mikrobizide Eigenschaften besitzen und zur Bekämpfung unerwünschter Mikroorganismen sowohl im Pflanzenschutz als auch im Materialschutz verwendbar sind.

Überraschenderweise zeigen die erfindungsgemäßen Difluormethylthiazolylcarboxanilide der Formel (I) eine wesentlich bessere fungizide Wirksamkeit als die konstitutionell ähnlichsten, vorbekannten Wirkstoffe gleicher Wirkungsrichtung.

Die erfindungsgemäßen Difluormethylthiazolylcarboxanilide sind durch die Formel (I) allgemein definiert.

Bevorzugt sind Difluormethylthiazolylcarboxanilide der Formel (I), in welcher
- R¹, R², R³, R⁴ und R⁵: unabhängig voneinander für Wasserstoff, Fluor, Chlor, Brom, Cyano, Nitro, Methyl, Ethyl, n- oder i-Propyl, n-, i-, s- oder t-Butyl, Methoxy, Ethoxy, Methylthio, Ethylthio, n- oder i-Propylthio, Cyclopropyl, Trifluor- methyl, Trichlormethyl, Trifluorethyl, Difluormethoxy, Trifluormethoxy, Difluorchlormethoxy, Trifluorethoxy, Difluormethylthio, Difluorchlormethyl- thio oder Trifluormethylthio stehen, mit der Maßgabe, dass R¹, R², R³, R⁴ und R⁵ nicht gleichzeitig für Wasserstoff stehen,
- R¹ und R² oder R² und R³: außerdem gemeinsam für gegebenenfalls durch Fluor, Chlor, Brom oder Methyl substituiertes Butadienylen stehen.

Besonders bevorzugt sind Difluormethylthiazolylcarboxanilide der Formel (I), in welcher
- R¹, R², R³, R⁴ und R⁵: unabhängig voneinander für Wasserstoff, Fluor, Chlor, Brom, Methyl, Methoxy, Methylthio, Trifluormethyl, Difluormethoxy, Trifluormeth- oxy, Difluormethylthio oder Trifluormethylthio stehen, mit der Maßgabe, dass R¹, R², R³, R⁴ und R⁵ nicht gleichzeitig für Wasserstoff stehen.

Weiterhin ganz besonders bevorzugt sind Difluormethylthiazolylcarboxanilide der Formel (I), in welcher
R¹, R², R⁴ und R⁵ jeweils für Wasserstoffstehen und
R³ die oben angegebenen Bedeutungen hat.

Weiterhin ganz besonders bevorzugt sind Difluormethylthiazolylcarboxanilide der Formel (I), in welcher
- R¹, R², R⁴ und R⁵: jeweils für Wasserstoff stehen und
- R³: für Fluor, Chlor, Brom, Methyl, Trifluormethyl, Trifluormethoxy oder Tri- fluormethylthio steht.

Weiterhin ganz besonders bevorzugt sind Difluormethylthiazolylcarboxanilide der Formel (I), in welcher
- R¹, R², R⁴ und R⁵: jeweils für Wasserstoff stehen und
- R³: für Cyano steht.

Weiterhin ganz besonders bevorzugt sind Difluormethylthiazolylcarboxanilide der Formel (I), in welcher
R², R⁴ und R⁵ für jeweils Wasserstoff stehen und
R¹ und R³ unabhängig voneinander die oben angegebenen Bedeutungen haben.

Weiterhin ganz besonders bevorzugt sind Difluormethylthiazolylcarboxanilide der Formel (I), in welcher
R², R⁴ und R⁵ für jeweils Wasserstoff stehen und
R¹ und R³ unabhängig voneinander für Fluor, Chlor, Brom, Methyl oder Trifluormethyl stehen.

Weiterhin ganz besonders bevorzugt sind Difluormethylthiazolylcarboxanilide der Formel (I), in welcher
R¹, R⁴ und R⁵ jeweils für Wasserstoff stehen und
R² und R³ unabhängig voneinander die oben angegebenen Bedeutungen haben.

Weiterhin ganz besonders bevorzugt sind Difluormethylthiazolylcarboxanilide der Formel (I), in welcher
R¹, R⁴ und R⁵ jeweils für Wasserstoff stehen und
R² und R³ unabhängig voneinander für Fluor, Chlor, Brom, Methyl oder Trifluormethyl stehen.

Weiterhin ganz besonders bevorzugt sind Difluormethylthiazolylcarboxanilide der Formel (I), in welcher
R¹, R³ und R⁵ jeweils für Wasserstoff stehen und
R² und R⁴ unabhängig voneinander die oben angegebenen Bedeutungen haben.

Weiterhin ganz besonders bevorzugt sind Difluormethylthiazolylcarboxanilide der Formel (I), in welcher
R¹, R³ und R⁵ jeweils für Wasserstoff stehen und
R² und R⁴ unabhängig voneinander für Fluor, Chlor, Brom, Methyl oder Trifluormethyl stehen.

Außerdem ganz besonders bevorzugt sind Difluormethylthiazolylcarboxanilide der Formel (I), in welcher R⁵ für Wasserstoff steht.

Verwendet man 2-Methyl-4-(difluormethyl)-1,3-thiazol-5-carbonylchlorid und 3'-Chlor-4'-fluor-1,1'-biphenyl-2-amin als Ausgangsstoffe sowie ein Base, so kann der Verlauf des erfindungsgemäßen Verfahrens a) durch folgende Reaktionsgleichung veranschaulicht werden:

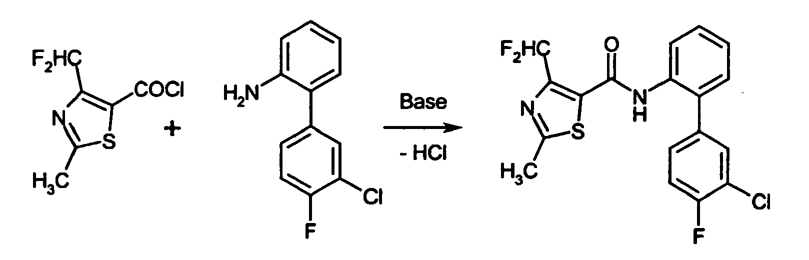

Die zur Durchführung des erfindungsgemäßen Verfahrens a) als Ausgangsstoffe benötigten Difluormethylthiazolylcarbonsäurehalogenide sind durch die Formel (II) allgemein definiert. In dieser Formel (II) steht X¹ bevorzugt für Chlor.

Die Difluormethylthiazolylcarbonsäurehalogenide der Formel (II) sind bekannt und/oder lassen sich nach bekannten Verfahren herstellen (vergleiche z.B. EP-A 0 276 177).

Die weiterhin zur Durchführung des erfindungsgemäßen Verfahrens a) als Ausgangsstoffe benötigten Aniline sind durch die Formel (III) allgemein definiert. In dieser Formel (III) stehen R¹, R², R³, R⁴ und R⁵ bevorzugt bzw. besonders bevorzugt für diejenigen Bedeutungen, die bereits im Zusammenhang mit der Beschreibung der erfindungsgemäßen Verbindungen der Formel (I) als bevorzugt bzw. als besonders bevorzugt für diese Reste angegeben wurden.

Die Anilin-Derivate der Formel (III) sind bekannt und/oder lassen sich nach bekannten Methoden herstellen (vgl. z.B. Bull. Korean Chem. Soc. 2000, 21, 165-166; Chem. Pharm. Bull. 1992, 40, 240-4; JP 09132567).

Verwendet man *N*-(2-Iodphenyl)-2-methyl-4-(difluormethyl)-1,3-thiazol-5-carboxamid und 3-Chlor-4-fluorphenylbomnsäure als Ausgangsstoffe sowie einen Katalystor und eine Base, so kann der Verlauf des erfindungsgemäßen Verfahrens b) kann durch folgende Reaktionsgleichung veranschaulicht werden:

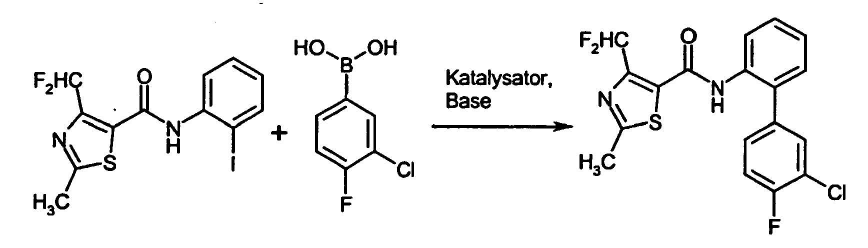

Die zur Durchführung des erfindungsgemäßen Verfahrens b) als Ausgangsstoffe benötigten Difluormethylthiazolylcarboxhalogenanilide sind durch die Formel (IV) allgemein definiert. In dieser Formel (IV) steht X² bevorzugt für Brom oder Iod.

Die Difluormethylthiazolylcarboxhalogenanilide der Formel (IV) sind noch nicht bekannt. Sie sind neue chemische Verbindungen und ebenfalls Gegenstand der vorliegenden Anmeldung. Sie werden erhalten, indem man
d) Difluormethylthiazolylcarbonsäurehalogenide der Formel (II) 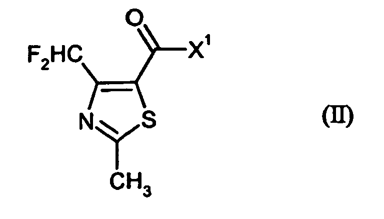 in welcher
   - X¹: für Halogen steht,
mit 2-Bromanilin oder 2-Iodanilin umsetzt.

Die zur Durchführung des erfindungsgemäBen Verfahrens d) als Ausgangsstoffe benötigten Difluormethylthiazolylcarbonsäurehalogenide der Formel (II) sind bereits weiter oben im Zusammenhang mit dem erfindungsgemäßen Verfahren a) beschrieben worden.

Die weiterhin zur Durchführung des erfindungsgemäßen Verfahrens d) als Ausgangsstoffe benötigten Stoffe 2-Bromanilin oder 2-Iodanilin sind bekannte Synthesechemikalien.

Die weiterhin zur Durchführung des erfindungsgemäßen Verfahrens b) als Ausgangsstoffe benötigten Boronsäurederivate sind durch die Formel (V) allgemein definiert. In dieser Formel (V) stehen R¹, R², R³, R⁴ und R⁵ bevorzugt bzw. besonders bevorzugt für diejenigen Bedeutungen, die bereits im Zusammenhang mit der Beschreibung der erfindungsgemäßen Verbindungen der Formel (I) als bevorzugt bzw. besonders bevorzugt für diese Reste angegeben wurden. G¹ und G² stehen bevorzugt jeweils für Wasserstoff oder zusammen für Tetramethylethylen.

Boronsäurederivate der Formel (V) sind bekannte Synthesechemikalien. Sie können auch unmittelbar vor der Reaktion direkt aus Halogenbenzolderivaten und Boronsäureestern hergestellt und ohne Aufarbeitung weiter umgesetzt werden (siehe auch die Herstellungsbeispiele).

Verwendet man 2-Methyl-*N*-[2-(4,4,5,5-tetramethyl-1,3,2-dioxaborolan-2-yl)phenyl]-4-(difluormethyl)-l,3-thiazol-5-carboxamid und 3-Chlor-4-fluorphenyl-trifluormethansulfonsäure als Ausgangsstoffe sowie einen Katalysator und eine Base, so kann der Verlauf des erfindungsgemäßen Verfahrens c) kann durch folgende Reaktionsgleichung veranschaulicht werden:

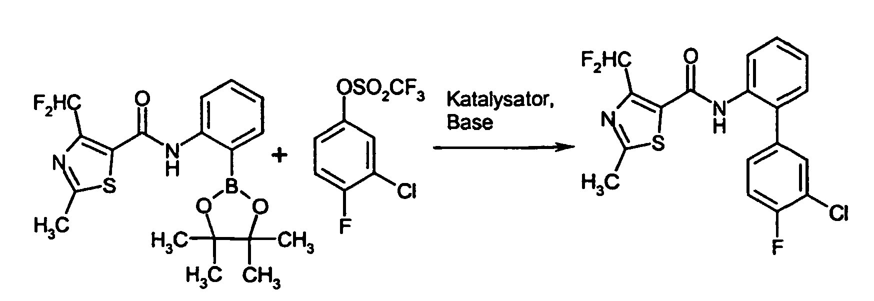

Die zur Durchführung des erfindungsgemäßen Verfahrens c) als Ausgangsstoffe benötigten Difluormethylthiazolylcarboxanilid-Boronsäure-Derivate sind durch die Formel (VI) allgemein definiert. In dieser Formel (VI) stehen G³ und G⁴ bevorzugt jeweils für Wasserstoff oder zusammen für Tetramethylethylen.

Die Difluormethylthiazolylcarboxanilid-Boronsäure-Derivate der Formel (VI) sind noch nicht bekannt. Sie sind neue chemische Verbindungen und ebenfalls Gegenstand der vorliegenden Anmeldung. Sie werden erhalten, indem man
e) Difluormethylthiazolylcarbonsäurehalogenide der Formel (II) 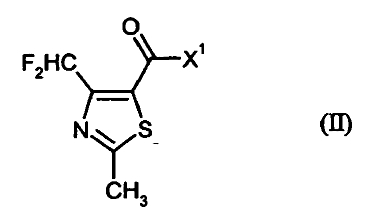 in welcher
   - X¹: für Halogen steht,
   mit Anilinboronsäurederivaten der Formel (VIII) 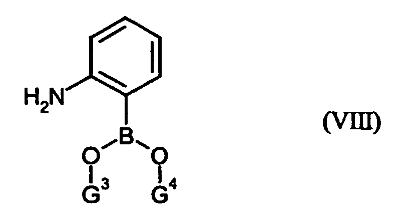 in welcher
   G³ und G⁴ die oben angegebenen Bedeutungen haben,
   gegebenenfalls in Gegenwart eines Säurebindemittels und gegebenenfalls in Gegenwart eines Verdünnungsmittels umsetzt.

Die zur Durchführung des erfindungsgemäßen Verfahrens e) als Ausgangsstoffe benötigten Difluormethylthiazolylcarbonsäurehalogenide der Formel (II) sind bereits weiter oben im Zusammenhang mit dem erfindungsgemäßen Verfahren a) beschrieben worden.

Die weiterhin zur Durchführung des erfindungsgemäßen Verfahrens e) als Ausgangsstoffe benötigten Anilinboronsäurederivate sind durch die Formel (VIII) allgemein definiert. In dieser Formel (VIII) stehen G³ und G⁴ bevorzugt jeweils für Wasserstoff oder zusammen für Tetramethylethylen.

Die zur Durchführung des erfindungsgemäßen Verfahrens e) als Ausgangsstoffe benötigten Anilinboronsäurederivate der Formel (VIII) sind bekannte Synthesechemikalien.

Die weiterhin zur Durchführung des erfindungsgemäßen Verfahrens c) als Ausgangsstoffe benötigten Halogenbenzolderivate sind durch die Formel (VII) allgemein definiert. In dieser Formel (VII) stehen R¹, R², R³, R⁴ und R⁵ bevorzugt bzw. besonders bevorzugt für diejenigen Bedeutungen, die bereits im Zusammenhang mit der Beschreibung der erfindungsgemäßen Verbindungen der Formel (I) als bevorzugt bzw. besonders bevorzugt für diese Reste angegeben wurden. X³ steht bevorzugt für Brom, Iod oder Trifluormethylsulfonyloxy.

Als Verdünnungsmittel zur Durchführung der erfindungsgemäßen Verfahrens a), d) und e) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; halogenierte Kohlenwasserstoffe, wie beispielsweise Chlorbenzol, Dichlorbenzol, Dichlormethan, Chloroform, Tetrachlormethan, Dichlorethan oder Trichlorethan; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol oder Amide, wie N,N-Dimethylfonnamid, N,N-Dimethylacetamid, N-Methylformanilid, N-Methylpyrrolidon oder Hexamethylphosphorsäuretriamid.

Die erfindungsgemäßen Verfahren a), d) und e) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, -carbonate oder -hydrogencarbonate, wie beispielsweise Natriumhydrid, Natriumamid, Natrium-methylat, Natrium-ethylat, Kalium-tert.-butylat, Natriumhydroxid, Kaliumhydroxid, Ammoniumhydroxid, Natriumacetat, Kaliumacetat, Calciumacetat, Ammoniumacetat, Natriumcarbonat, Kaliumcarbonat, Kaliumhydrogencarbonat, Natriumhydrogencarbonat oder Caesiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren a), d) und e) in einem größeren Bereich variiert werden. Im allgemeinen arbeitet man bei Temperaturen von 0°C bis 150°C, vorzugsweise bei Temperaturen von 20°C bis 110°C.

Zur Durchführung des erfindungsgemäßen Verfahrens a) zur Herstellung der Verbindungen der Formel (I) setzt man pro Mol des Difluormethylthiazolylcarbonsäurehalogenides der Formel (II) im allgemeinen 0,2 bis 5 Mol, vorzugsweise 0,5 bis 2 Mol an Anilinderivat der Formel (III) ein.

Zur Durchführung des erfindungsgemäßen Verfahrens d) zur Herstellung der Verbindungen der Formel (III) setzt man pro Mol des Difluormethylthiazolylcarbonsäurehalogenides der Formel (II) im allgemeinen 0,2 bis 5 Mol, vorzugsweise 0,5 bis 2 Mol an 2-Bromanilin oder 2-Iodanilin ein.

Zur Durchführung des erfindungsgemäßen Verfahrens e) zur Herstellung der Verbindungen der Formel (VI) setzt man pro Mol des Difluormethylthiazolylcarbonsäurehalogenides der Formel (II) im allgemeinen 0,2 bis 5 Mol, vorzugsweise 0,5 bis 2 Mol an Anilinboronsäurederivat der Formel (VIII) ein.

Als Verdünnungsmittel zur Durchführung der erfindungsgemäßen Verfahren b) und c) kommen alle inerten organischen Lösungsmittel in Betracht. Hierzu gehören vorzugsweise aliphatische, alicyclische oder aromatische Kohlenwasserstoffe, wie beispielsweise Petrolether, Hexan, Heptan, Cyclohexan, Methylcyclohexan, Benzol, Toluol, Xylol oder Decalin; Ether, wie Diethylether, Diisopropylether, Methyl-t-butylether, Methyl-t-Amylether, Dioxan, Tetrahydrofuran, 1,2-Dimethoxyethan, 1,2-Diethoxyethan oder Anisol; Nitrile, wie Acetonitril, Propionitril, n- oder i-Butyronitril oder Benzonitril; Amide, wie N,N-Dimethylformamid, N,N-Dimethylacetamid, N-Methylformanilid, N-Methylpyrrolidon oder Hexamethylphosphorsäuretriamid; Ester wie Essigsäuremethylester oder Essigsäureethylester, Sulfoxide, wie Dimethylsulfoxid; Sulfone, wie Sulfolan; Alkohol, wie Methanol, Ethanol, n- oder i-Propanol, n-, i-, sek- oder tert-Butanol, Ethandiol, Propan-1,2-diol, Ethoxyethanol, Methoxyethanol, Diethylenglykolmonomethylether, Diethylenglykolmonoethylether, deren Gemische mit Wasser oder reines Wasser.

Die Reaktionstemperaturen können bei der Durchführung der erfindungsgemäßen Verfahrens b) und c) in einem größeren Bereich variiert werden. Im allgemeinen arbeitet man bei Temperaturen von 0°C bis 150°C, vorzugsweise bei Temperaturen von 20°C bis 110°C.

Die erfindungsgemäßen Verfahren b) und c) werden gegebenenfalls in Gegenwart eines geeigneten Säureakzeptors durchgeführt. Als solche kommen alle üblichen anorganischen oder organischen Basen infrage. Hierzu gehören vorzugsweise Erdalkalimetall- oder Alkalimetallhydride, -hydroxide, -amide, -alkoholate, -acetate, fluoride, phosphate, -carbonate oder -hydrogencarbonate, wie beispielsweise Natriumhydrid, Natriumamid, Lithiumdiisopropylamid, Natrium-methylat, Natrium-ethylat, Kalium-tert.-butylat, Natriumhydroxid, Kaliumhydroxid, Natriumacetat, Natriumphosphat, Kaliumphosphat, Kaliumfluorid, Cäsiumfluorid, Natriumcarbonat, Kaliumcarbonat, Kaliumhydrogencarbonat, Natriumhydrogencarbonat oder Cäsiumcarbonat, sowie tertiäre Amine, wie Trimethylamin, Triethylamin, Tributylamin, N,N-Dimethylanilin, N,N-Dimethyl-benzylamin, Pyridin, N-Methylpiperidin, N-Methylmorpholin, N,N-Dimethylaminopyridin, Diazabicyclooctan (DABCO), Diazabicyclononen (DBN) oder Diazabicycloundecen (DBU).

Die erfindungsgemäßen Verfahren b) und c) werden in Gegenwart eines Katalysators, wie beispielsweise eines Palladiumsalzes oder -komplexes, durchgeführt. Hierzu kommen vorzugsweise Palladiumchlorid, Palladiumacetat, Tetrakis-(triphenylphosphin)-Palladium, Bis-(triphenylphosphin)-Palladiumdichlorid oder 1,1'-Bis(di-phenylphosphino)ferrocenpalladium(II)chlorid infrage.

Es kann auch ein Palladiumkomplex in der Reaktionsmischung erzeugt werden, wenn man ein Palladiumsalz und ein Komplexligand, wie beispielsweise Triethylphosphan, Tri-tert-butylphosphan, Tricyclohexylphosphan, 2-(Dicyclohexylphosphan)-biphenyl, 2-(Di-tert-butylphosphan)-biphenyl, 2-(Dicyclohexylphosphan)-2'-(N,N-dimethylamino)-biphenyl, Triphenylphosphan, Tris-(o-Tolyl)-phosphan, Natrium-3-(diphenylphosphino)benzolsulfonat, Tris-2-(Methoxyphenyl)-phosphan, 2,2'-Bis-(diphenylphosphan)-1,1'-binaphthyl, 1,4-Bis-(diphenylphosphan)-butan, 1,2-Bis-(diphenylphosphan)-ethan, 1,4-Bis-(dicyclohexylphosphan)-butan, 1,2-Bis-(dicyclohexylphosphan)-ethan, 2-(Dicyclohexylphosphan)-2'-(N,N-dimethylamino)-biphenyl, Bis(diphenylphosphino)ferrocen oder Tris-(2,4-tert-butylphenyl)-phosphit getrennt zur Reaktion zugibt.

Zur Durchführung des erfindungsgemäßen Verfahrens b) zur Herstellung der Verbindungen der Formel (I) setzt man pro Mol des Difluormethylthiazolylcarboxhalogenanilide der Formel (IV) im allgemeinen 1 bis 15 Mol, vorzugsweise 2 bis 8 Mol an Boronsäurederivat der Formel (V) ein.

Zur Durchführung des erfindungsgemäßen Verfahrens c) zur Herstellung der Verbindungen der Formel (I) setzt man pro Mol des Difluormethylthiazolylcarboxanilid-Boronsäure-Derivates der Formel (VI) im allgemeinen 1 bis 15 Mol, vorzugsweise 2 bis 8 Mol an Halogenbenzolderivat der Formel (VII) ein.

Die erfindungsgemäßen Verfahren a), b), c) und d) werden im allgemeinen unter Normaldruck durchgeführt. Es ist jedoch auch möglich, unter erhöhtem oder vermindertem Druck - im allgemeinen zwischen 0,1 bar und 10 bar - zu arbeiten.

Die erfindungsgemäßen Stoffe weisen eine starke mikrobizide Wirkung auf und können zur Bekämpfung von unerwünschten Mikroorganismen, wie Fungi und Bakterien, im Pflanzenschutz und im Materialschutz eingesetzt werden.

Fungizide lassen sich Pflanzenschutz zur Bekämpfung von Plasmodiophoromycetes, Oomycetes, Chytridiomycetes, Zygomycetes, Ascomycetes, Basidiomycetes und Deuteromycetes einsetzen.

Bakterizide lassen sich im Pflanzenschutz zur Bekämpfung von Pseudomonadaceae, Rhizobiaceae, Enterobacteriaceae, Corynebacteriaceae und Streptomycetaceae einsetzen.

Beispielhaft aber nicht begrenzend seien einige Erreger von pilzlichen und bakteriellen Erkrankungen, die unter die oben aufgezählten Oberbegriffe fallen, genannt: Xanthomonas-Arten, wie beispielsweise Xanthomonas campestris pv. oryzae; Pseudomonas-Arten, wie beispielsweise Pseudomonas syringae pv. lachrymans; Erwinia-Arten, wie beispielsweise Erwinia amylovora; Pythium-Arten, wie beispielsweise Pythium ultimum; Phytophthora-Arten, wie beispielsweise Phytophthora infestans; Pseudoperonospora-Arten, wie beispielsweise Pseudoperonospora humuli oder Pseudoperonospora cubensis; Plasmopara-Arten, wie beispielsweise Plasmopara viticola; Bremia-Arten, wie beispielsweise Bremia lactucae; Peronospora-Arten, wie beispielsweise Peronospora pisi oder P. brassicae; Erysiphe-Arten, wie beispielsweise Erysiphe graminis; Sphaerotheca-Arten, wie beispielsweise Sphaerotheca fuliginea; Podosphaera-Arten, wie beispielsweise Podosphaera leucotricha; Venturia-Arten, wie beispielsweise Venturia inaequalis; Pyrenophora-Arten, wie beispielsweise Pyrenophora teres oder P. graminea (Konidienform: Drechslera, Syn: Helminthosporium); Cochliobolus-Arten, wie beispielsweise Cochliobolus sativus (Konidienform: Drechslera, Syn: Helminthosporium); Uromyces-Arten, wie beispielsweise Uromyces appendiculatus; Puccinia-Arten, wie beispielsweise Puccinia recondita; Sclerotinia-Arten, wie beispielsweise Sclerotinia sclerotiorum; Tilletia-Arten, wie beispielsweise Tilletia caries; Ustilago-Arten, wie beispielsweise Ustilago nuda oder Ustilago avenae; Pellicularia-Arten, wie beispielsweise Pellicularia sasakii; Pyricularia-Arten, wie beispielsweise Pyricularia oryzae; Fusarium-Arten, wie beispielsweise Fusarium culmorum; Botrytis-Arten, wie beispielsweise Botrytis cinerea; Septoria-Arten, wie beispielsweise Septoria nodorum; Leptosphaeria-Arten, wie beispielsweise Leptosphaeria nodorum; Cercospora-Arten, wie beispielsweise Cercospora canescens; Alternaria-Arten, wie beispielsweise Alternaria brassicae; Pseudocercosporella-Arten, wie beispielsweise Pseudocercosporella herpotrichoides.

Die erfindungsgemäßen Wirkstoffe weisen auch eine starke stärkende Wirkung in Pflanzen auf. Sie eignen sich daher zur Mobilisierung pflanzeneigener Abwehrkräfte gegen Befall durch unerwünschte Mikroorganismen.

Unter pflanzenstärkenden (resistenzinduzierenden) Stoffen sind im vorliegenden Zusammenhang solche Substanzen zu verstehen, die in der Lage sind, das Abwehrsystem von Pflanzen so zu stimulieren, dass die behandelten Pflanzen bei nachfolgender Inokulation mit unerwünschten Mikroorganismen weitgehende Resistenz gegen diese Mikroorganismen entfalten.

Unter unerwünschten Mikroorganismen sind im vorliegenden Fall phytopathogene Pilze, Bakterien und Viren zu verstehen. Die erfindungsgemäßen Stoffe können also eingesetzt werden, um Pflanzen innerhalb eines gewissen Zeitraumes nach der Behandlung gegen den Befall durch die genannten Schaderreger zu schützen. Der Zeitraum, innerhalb dessen Schutz herbeigeführt wird, erstreckt sich im allgemeinen von 1 bis 10 Tage, vorzugsweise 1 bis 7 Tage nach der Behandlung der Pflanzen mit den Wirkstoffen.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut, und des Bodens.

Die erfindungsgemäßen Wirkstoffe eignen sich auch zur Steigerung des Ernteertrages. Sie sind außerdem mindertoxisch und weisen eine gute Pflanzenverträglichkeit auf.

Die erfindungsgemäßen Wirkstoffe können gegebenenfalls in bestimmten Konzentrationen und Aufwandmengen auch als Herbizide, zur Beeinflussung des Pflanzenwachstums, sowie zur Bekämpfung von tierischen Schädlingen verwendet werden. Sie lassen sich gegebenenfalls auch als Zwischen- und Vorprodukte für die Synthese weiterer Wirkstoffe einsetzen.

Erfindungsgemäß können alle Pflanzen und Pflanzenteile behandelt werden. Unter Pflanzen werden hierbei alle Pflanzen und Pflanzenpopulationen verstanden, wie erwünschte und unerwünschte Wildpflanzen oder Kulturpflanzen (einschließlich natürlich vorkommender Kulturpflanzen). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen, wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Wirkstoffen erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen und bei Vermehrungsmaterial, insbesondere bei Samen, weiterhin durch ein- oder mehrschichtiges Umhüllen.

Im Materialschutz lassen sich die erfindungsgemäßen Stoffe zum Schutz von technischen Materialien gegen Befall und Zerstörung durch unerwünschte Mikroorganismen einsetzen.

Unter technischen Materialien sind im vorliegenden Zusammenhang nichtlebende Materialien zu verstehen, die für die Verwendung in der Technik zubereitet worden sind. Beispielsweise können technische Materialien, die durch erfindungsgemäße Wirkstoffe vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Wärmeübertragungsflüssigkeiten genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirken die erfindungsgemäßen Wirkstoffe gegen Pilze, insbesondere Schimmelpilze, holzverfärbende und holzzerstörende Pilze (Basidiomyceten) sowie gegen Schleimorganismen und Algen.

Es seien beispielsweise Mikroorganismen der folgenden Gattungen genannt: Alternaria, wie Alternaria tenuis, Aspergillus, wie Aspergillus niger, Chaetomium, wie Chaetomium globosum, Coniophora, wie Coniophora puetana, Lentinus, wie Lentinus tigrinus, Penicillium, wie Penicillium glaucum, Polyporus, wie Polyporus versicolor, Aureobasidium, wie Aureobasidium pullulans, Sclerophoma, wie Sclerophoma pityophila, Trichoderma, wie Trichoderma viride, Escherichia, wie Escherichia coli, Pseudomonas, wie Pseudomonas aeruginosa, Staphylococcus, wie Staphylococcus aureus.

Die Wirkstoffe können in Abhängigkeit von ihren jeweiligen physikalischen und/oder chemischen Eigenschaften in die üblichen Formulierungen überführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser. Mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid. Als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate. Als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnussschalen, Maiskolben und Tabakstängel. Als Emulgier und/oder schaumerzeugende Mittel kommen in Frage: z.B. nichtionogene und anionische Emulgatoren, wie Polyoxyethylen-Fettsäureester, Polyoxyethylen-Fettallcoholether, z.B. Alkylarylpolyglycolether, Alkylsulfonate, Alkylsulfate, Arylsulfonate sowie Eiweißhydrolysate. Als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe, wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen. In vielen Fällen erhält man dabei synergistische Effekte, d.h. die Wirksamkeit der Mischung ist größer als die Wirksamkeit der Einzelkomponenten.

Als Mischpartner kommen zum Beispiel folgende Verbindungen in Frage:
Fungizide:
   Aldimorph, Ampropylfos, Ampropylfos-Kalium, Andoprim, Anilazin, Azaconazol, Azoxystrobin,
   Benalaxyl; Benodanil, Benomyl, Benzamacril, Benzamacryl-isobutyl, Bialaphos, Binapacryl, Biphenyl, Bitertanol, Blasticidin-S, Bromuconazol, Bupirimat, Buthiobat, Calciumpolysulfid, Carpropamid, Capsimycin, Captafol, Captan, Carbendazim, Carboxin, Carvon, Chinomethionat (Quinomethionat), Chlobenthiazon, Chlorfenazol, Chloroneb, Chloropicrin, Chlorothalonil, Chlozolinat, Clozylacon, Cufraneb, Cymoxanil, Cyproconazol, Cyprodinil, Cyprofuram,
   Debacarb, Dichlorophen, Diclobutrazol, Diclofluanid, Diclomezin, Dicloran, Diethofencarb, Difenoconazol, Dimethirimol, Dimethomorph, Diniconazol, Diniconazol-M, Dinocap, Diphenylamin, Dipyrithione, Ditalimfos, Dithianon, Dodemorph, Dodine, Drazoxolon,
   Ediphenphos, Epoxiconazol, Etaconazol, Ethirimol, Etridiazol,
   Famoxadon, Fenapanil, Fenarimol, Fenbuconazol, Fenfuram, Fenhexamid, Fenitropan, Fenpicionil, Fenpropidin, Fenpropimorph, Fentinacetat, Fentinhydroxyd, Ferbam, Ferimzon, Fluazinam, Flumetover, Fluoromid, Fluquinconazol, Flurprimidol, Flusilazol, Flusulfamid, Flutolanil, Flutriafol, Folpet, Fosetyl-Alminium, Fosetyl-Natrium, Fthalid, Fuberidazol, Furalaxyl, Furametpyr, Furcarbonil, Furconazol, Furconazol-cis, Furmecyclox,
   Guazatin, Hexachlorobenzol, Hexaconazol, Hymexazol,
   Imazalil, Imibenconazol, Iminoctadin, Iminoctadinealbesilat, Iminoctadinetriacetat, Iodocarb, Ipconazol, Iprobenfos (IBP), Iprodione, Iprovalicarb, Irumamycin, Isoprothiolan, Isovaledione,
   Kasugamycin, Kresoxim-methyl, Kupfer-Zubereitungen, wie: Kupferhydroxid, Kupfernaphthenat, Kupferoxychlorid, Kupfersulfat, Kupferoxid, Oxin-Kupfer und Bordeaux-Mischung,
   Mancopper, Mancozeb, Maneb, Meferimzone, Mepanipyrim, Mepronil, Metalaxyl, Metconazol, Methasulfocarb, Methfuroxam, Metiram, Metomeclam, Metsulfovax, Mildiomycin, Myclobutanil, Myclozolin,
   Nickel-dimethyldithiocarbamat, Nitrothal-isopropyl, Nuarimol,
   Ofurace, Oxadixyl, Oxamocarb, Oxolinicacid, Oxycarboxim, Oxyfenthiin,
   Paclobutrazol, Pefurazoat, Penconazol, Pencycuron, Phosdiphen, Picoxystrobin, Pimaricin, Piperalin, Polyoxin, Polyoxorim, Probenazol, Prochloraz, Procymidon, Propamocarb, Propanosine-Natrium, Propiconazol, Propineb, Pyraclostrobin, Pyrazophos, Pyrifenox, Pyrimethanil, Pyroquilon, Pyroxyfur,
   Quinconazol, Quintozen (PCNB), Quinoxyfen,
   Schwefel und Schwefel-Zubereitungen, Spiroxamine,
   Tebuconazol, Tecloftalam, Tecnazen, Tetcyclacis, Tetraconazol, Thiabendazol, Thicyofen, Thifluzamide, Thiophanate-methyl, Thiram, Tioxymid, Tolclofos-methyl, Tolylfluanid, Triadimefon, Triadimenol, Triazbutil, Triazoxid, Trichlamid, Tricyclazol, Tridemorph, Trifloxystrobin, Triflumizol, Triforin, Triticonazol,
   Uniconazol, Validamycin A, Vinclozolin, Viniconazol,
   Zarilamid, Zineb, Ziram sowie
   Dagger G, OK-8705, OK-8801,
   α-(1,1-Dimethylethyl)-ß-(2-phenoxyethyl)-1H-1,2,4-triazol-1-ethanol,
   α-(2,4-Dichlorphenyl)-ß-fluor-B-propyl-1H-1,2,4-triazol-1-ethanol,
   α-(2,4-Dichlorphenyl)-ß-methoxy-α-methyl-1H-1,2,4-triazol-1-ethanol,
   α-(5-Methyl-1,3-dioxan-5-yl)-ß-[[4-(trifluormethyl)-phenyl]-methylen]-1H-1,2,4-triazol-1-ethanol,
   (5RS,6P,S)-6-Hydroxy-2,2,7,7-tetramethyl-5-(1H-1,2,4-triazol-1-yl)-3-octanon,
   (E)-α-(Methoxyimino)-N-methyl-2-phenoxy-phenylacetamid,
   1-(2,4-Dichlorphenyl)-2-(1H-1,2,4-triazol-1-yl)-ethanon-O-(phenylmethyl)-oxim,
   1-(2-Methyl-1-naphthalenyl)-1H-pyrrol-2,5-dion,
   1-(3,5-Dichlorphenyl)-3-(2-propenyl)-2,5-pyrrolidindion,
   1-[(Diiodmethyl)-sulfonyl]-4-methyl-benzol,
   1-[[2-(2,4-Dichlotphenyl)-1,3-dioxolan-2-yl]-methyl]-1H-imidazol,
   1-[[2-(4-Chlorphenyl)-3-phenyloxiranyl]-methyl]-1H-1,2,4-triazol,
   1-[1-[2-[(2,4-Dichlorphenyl)-methoxy]-phenyl]-ethenyl]-1H-imidazol,
   1-Methyl-5-nonyl-2-(phenylmethyl)-3-pyrrolidinol,
   2',6'-Dibrom-2-methyl-4'-trifluormethoxy-4'-trifluor-methyl-1,3-thiazol-5-carboxanilid,
   2,6-Dichlor-5-(methylthio)-4-pyrimidinyl-thiocyanat,
   2,6-Dichlor-N-(4-trifluormethylbenzyl)-benzamid,
   2,6-Dichlor-N-[[4-(trifluormethyl)-phenyl]-methyl]-benzamid,
   2-(2,3,3-Triiod-2-propenyl)-2H-tetrazol,
   2-[(1-Methylethyl)-sulfonyl]-5-(trichlormethyl)-1,3,4-thiadiazol,
   2-[[6-Deoxy-4-O-(4-O-methyl-ß-D-glycopyranosyl)-α-D-glucopyranosyl]-amino]-4-methoxy-1H-pyrrolo[2,3-d]pyrimidin-5-carbonitril,
   2-Aminobutan,
   2-Brom-2-(brommethyl)-pentandinitril,
   2-Chlor-N-(2,3-dihydro-1,1,3-trimethyl-1H-inden-4-yl)-3-pyridincarboxamid,
   2-Chlor-N-(2,6-dimethylphenyl)-N-(isothiocyanatomethyl)-acetamid, 2-Phenylphenol(OPP),
   3,4-Dichlor-1-[4-(difluormethoxy)-phenyl]-1H-pyrrol-2,5-dion,
   3,5-Dichlor-N-[cyan[(1-methyl-2-propynyl)-oxy]-methyl]-benzamid,
   3-(1,1-Dimethylpropyl-1-oxo-1H-inden-2-carbonitril,
   3-[2-(4-Chlorphenyl)-5-ethoxy-3-isoxazolidinyl]-pyridin,
   4-Chlor-2-cyan-N,N-dimethyl-5-(4-methylphenyl)-1H-imidazol-1-sulfonamid, 4-Methyl-tetrazolo[1,5-a]quinazolin-5(4H)-on,
   8-Hydroxychinolinsulfat,
   9H-Xanthen-9-carbonsäure-2-[(phenylamino)-carbonyl]-hydrazid,
   bis-(1-Methylethyl)-3-methyl-4-[(3-methylbenzoyl)-oxy]-2,5-thiophendicarboxylat,
   cis-1-(4-Chlorphenyl)-2-(1H-1,2,4-triazol-1-yl)-cycloheptanol,
   cis-4-[3-[4-(1,1-Dimethylpropyl)-phenyl-2-methylpropyl]-2,6-dimethyl-morpholinhydrochlorid,
   Ethyl-[(4-chlorphenyl)-azo]-cyanoacetat,
   Kaliumhydrogencarbonat,
   Methantetrathiol-Natriumsalz,
   Methyl-1-(2,3-dihydro-2,2-dimethyl-1H-inden-1-yl)-1H-imidazol-5-carboxylat,
   Methyl-N-(2,6-dimethylphenyl)-N-(5-isoxazolylcarbonyl)-DL-alaninat,
   Methyl-N-(chloracetyl)-N-(2,6-dimethylphenyl)-DL-alaninat,
   N-(2,6-Dimethylphenyl)-2-methoxy-N-(tetrahydro-2-oxo-3-furanyl)-acetamid,
   N-(2,6-Dimethylphenyl)-2-methoxy N-(tetrahydro-2-oxo-3-thienyl)-acetamid,
   N-(2-Chlor-4-nitrophenyl)-4-methyl-3-nitro-benzolsulfonamid,
   N-(4-Cyclohexylphenyl)-1,4,5,6-tetrahydro-2-pyrimidinamin,
   N-(4-Hexylphenyl)-1,4,5,6-tetrahydro-2-pyrimidinamin,
   N-(5-Chlor-2-methylphenyl)-2-methoxy-N-(2-oxo-3-oxazolidinyl)-acetamid,
   N-(6-Methoxy)-3-pyridinyl)-cyclopropancarboxamid,
   N-[2,2,2-Trichlor-1-[(chloracetyl)-amino]-ethyl]-benzamid,
   N-[3-Chlor-4,5-bis-(2-propinyloxy)-phenyl]-N'-methoxy-methanimidamid,
   N-Formyl-N-hydroxy-DL-alanin-Natriumsalz,
   O,O-Diethyl-[2-(dipropylamino)-2-oxoethyl]-ethylphosphoramidothioat,
   O-Methyl-S-phenyl-phenylpropylphosphoramidothioate,
   S-Methyl-1,2,3-benzothiadiazol-7-carbothioat,
   spiro[2H]-1-Benzopyran-2,1'(3'H)-isobenzofuran]-3'-on,
   4-[3,4-Dimethoxyphenyl)-3-(4-fluorphenyl)-acryloyl]-morpholin
Bakterizide:
   Bronopol, Dichlorophen, Nitrapyrin, Nickel-dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.
Insektizide / Akarizide / Nematizide:
   Abamectin, Acephate, Acetamiprid, Acrinathrin, Alanycarb, Aldicarb, Aldoxycarb, Alpha-cypermethrin, Alphamethrin, Amitraz, Avermectin, AZ 60541, Azadirachtin, Azamethiphos, Azinphos A, Azinphos M, Azocyclotin,
   Bacillus popilliae, Bacillus sphaericus, Bacillus subtilis, Bacillus thuringiensis, Baculoviren, Beauveria bassiana, Beauveria tenella, Bendiocarb, Benfuracarb, Bensultap, Benzoximate, Betacyfluthrin, Bifenazate, Bifenthrin, Bioethanomethrin, Biopermethrin, Bistrifluron, BPMC, Bromophos A, Bufencarb, Buprofezin, Butathiofos, Butocarboxim, Butylpyridaben,
   Cadusafos, Carbaryl, Carbofuran, Carbophenothion, Carbosulfan, Cartap, Chloethocarb, Chlorethoxyfos, Chlorfenapyr, Chlorfenvinphos, Chlorfluazuron, Chlormephos, Chlorpyrifos, Chlorpyrifos M, Chlovaporthrin, Chromafenozide, Cis-Resmethrin, Cispermethrin, Clocythrin, Cloethocarb, Clofentezine, Clothianidine, Cyanophos, Cycloprene, Cycloprothrin, Cyfluthrin, Cyhalothrin, Cyhexatin, Cypermethrin, Cyromazine,
   Deltamethrin, Demeton M, Demeton S, Demeton-S-methyl, Diafenthiuron, Diazinon, Dichlorvos, Dicofol, Diflubenzuron, Dimethoat, Dimethylvinphos, Diofenolan, Disulfoton, Docusat-sodium, Dofenapyn,
   Eflusilanate, Emamectin, Empenthrin, Endosulfan, Entomopfthora spp., Esfenvalerate, Ethiofencarb, Ethion, Ethoprophos, Etofenprox, Etoxazole, Etrimfos,
   Fenamiphos, Fenazaquin, Fenbutatin oxide, Fenitrothion, Fenothiocarb, Fenoxacrim, Fenoxycarb, Fenpropathrin, Fenpyrad, Fenpyrithrin, Fenpyroximate, Fenvalerate, Fipronil, Fluazuron, Flubrocythrinate, Flucycloxuron, Flucythrinate, Flufenoxuron, Flumethrin, Flutenzine, Fluvalinate, Fonophos, Fosmethilan, Fosthiazate, Fubfenprox, Furathiocarb,
   Granuloseviren
   Halofenozide, HCH, Heptenophos, Hexaflumuron, Hexythiazox, Hydroprene,
   Imidacloprid, Indoxacarb, Isazofos, Isofenphos, Isoxathion, Ivermectin,
   Kempolyederviren, Lambda-cyhalothrin, Lufenuron
   Malathion, Mecarbam, Metaldehyd, Methamidophos, Metharhizium anisopliae, Metharhizium flavoviride, Methidathion, Methiocarb, Methoprene, Methomyl, Methoxyfenozide, Metolcarb, Metoxadiazone, Mevinphos, Milbemectin, Milbemycin, Monocrotophos,
   Naled, Nitenpyram, Nithiazine, Novaluron, Omethoat, Oxamyl, Oxydemethon M, Paecilomyces fumosoroseus, Parathion A, Parathion M, Permethrin, Phenthoat, Phorat, Phosalone, Phosmet, Phosphamidon, Phoxim, Pirimicarb, Pirimiphos A,
   Pirimiphos M, Profenofos, Promecarb, Propargite, Propoxur, Prothiofos, Prothoat, Pymetrozine, Pyraclofos, Pyresmethrin, Pyrethrum, Pyridaben, Pyridathion, Pyrimidifen, Pyriproxyfen, Quinalphos, Ribavirin,
   Salithion, Sebufos, Silafluofen, Spinosad, Spirodiclofen, Sulfotep, Sulprofos,
   Tau-fluvalinate, Tebufenozide, Tebufenpyrad, Tebupirimiphos, Teflubenzuron, Tefluthrin, Temephos, Temivinphos, Terbufos, Tetrachlorvinphos, Tetradifon, Thetacypermethrin, Thiacloprid, Thiamethoxam, Thiapronil, Thiatriphos, Thiocyclam hydrogen oxalate, Thiodicarb, Thiofanox, Thuringiensin, Tralocythrin, Tralomethrin, Triarathene, Triazamate, Triazophos, Triazuron, Trichlophenidine, Trichlorfon, Triflumuron, Trimethacarb,
   Vamidothion, Vaniliprole, Verticillium lecanii,
   YI 5302, Zeta-cypermethrin, Zolaprofos
   (1R-cis)-[5-(Phenylmethyl)-3-furanyl]-methyl-3-[(dihydro-2-oxo-3(2H)-furanyliden)-methyl]-2,2-dimethylcyclopropancarboxylat
   (3-Phenoxyphenyl)-methyl- 2,2,3,3-tetramethylcyclopropanecarboxylat 1-[(2-Chlor-5-thiazolyl)methyl]tetrahydro-3,5-dimethyl-N-nitro-1,3,5-triazin-2(1H)-imin
   2-(2-Chlor-6-fluorphenyl)-4-[4-(1,1-dimethylethyl)phenyl]-4,5-dihydro-oxazol 2-(Acetlyoxy)-3-dodecyl-1,4-naphthalindion
   2-Chlor-N-[[[4-(1-phenylethoxy)-phenyl]-amino]-carbonyl]-benzamid 2-Chlor-N-[[[4-(2,2-dichlor-1,1-difluorethoxy)-phenyl]-amino]-carbonyl]-benzamid 3-Methylphenyl-propylcarbamat
   4-[4-(4-Ethoxyphenyl)-4-methylpentyl]-1-fluor-2-phenoxy-benzol 4-Chlor-2-(1,1-dimethylethyl)-5-[[2-(2,6-dimethyl-4-phenoxyphenoxy)ethyl]thio]-3(2H)-pyridazinon
   4-Chlor-2-(2-chlor-2-methylpropyl)-5-[(6-iod-3-pyridinyl)methoxy]-3(2H)-pyridazinon
   4-Chlor-5-[(6-chlor-3-pyridinyl)methoxy]-2-(3,4-dichlorphenyl)-3(2H)-pyridazinon Bacillus thuringiensis strain EG-2348
   Benzoesäure [2-benzoyl-1-(1,1-dimethylethyl)-hydrazid
   Butansäure 2,2-dimethyl-3-(2,4-dichlorphenyl)-2-oxo-1-oxaspiro[4.5]dec-3-en-4-yl-ester
   [3-[(6-Chlor-3-pyridinyl)methyl]-2-thiazolidinyliden]-cyanamid
   Dihydro-2-(nitromethylen)-2H-1,3-thiazine-3(4H)-carboxaldehyd
   Ethyl-[2-[[1,6-dihydro-6-oxo-1-(phenylmethyl)-4-pyridazinyl]oxy]ethyl]-carbamat N-(3,4,4-Trifluor-1-oxo-3-butenyl)-glycin
   N-(4-Chlorphenyl)-3-[4-(difluormethoxy)phenyl]-4,5-dihydro-4-phenyl-1H-pyrazol-1-carboxamid
   N-[(2-Chlor-5-thiazolyl)methyl]-N'-methyl-N"-nitro-guanidin
   N-Methyl-N'-(1-methyl-2-propenyl)-1,2-hydrazindicarbothioamid
   N-Methyl-N'-2-propenyl-1,2-hydrazindicarbothioamid
   O,O-Diethyl-[2-(dipropylamino)-2-oxoethyl]-ethylphosphoramidothioat
   N-Cyanomethyl-4-trifluormethyl-nicotinamid
   3,5-Dichlor-1-(3,3-dichlor-2-propenyloxy)-4-[3-(5-trifluormethylpyridin-2-yloxy)-propoxy]-benzol

Auch eine Mischung mit anderen bekannten Wirkstoffen, wie Herbiziden oder mit Düngemitteln und Wachstumsregulatoren ist möglich.

Darüber hinaus weisen die erfindungsgemäßen Verbindungen der Formel (I) auch sehr gute antimykotische Wirkungen auf. Sie besitzen ein sehr breites antimykotisches Wirkungsspektrum, insbesondere gegen Dermatophyten und Sprosspilze, Schimmel und diphasische Pilze (z.B. gegen Candida-Spezies wie Candida albicans, Candida glabrata) sowie Epidermophyton floccosum, Aspergillus-Spezies wie Aspergillus niger und Aspergillus fumigatus, Trichophyton-Spezies wie Trichophyton mentagrophytes, Microsporon-Spezies wie Microsporon canis und audouinii. Die Aufzählung dieser Pilze stellt keinesfalls eine Beschränkung des erfassbaren mykotischen Spektrums dar, sondern hat nur erläuternden Charakter.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Wirkstoffe als Fungizide können die Aufwandmengen je nach Applikationsart innerhalb eines größeren Bereiches variiert werden. Bei der Behandlung von Pflanzenteilen liegen die Aufwandmengen an Wirkstoff im allgemeinen zwischen 0,1 und 10.000 g/ha, vorzugsweise zwischen 10 und 1.000 g/ha. Bei der Saatgutbehandlung liegen die Aufwandmengen an Wirkstoff im allgemeinen zwischen 0,001 und 50 g pro Kilogramm Saatgut, vorzugsweise zwischen 0,01 und 10 g pro Kilogramm Saatgut. Bei der Behandlung des Bodens liegen die Aufwandmengen an Wirkstoff im allgemeinen zwischen 0,1 und 10.000 g/ha, vorzugsweise zwischen 1 und 5.000 g/ha.

Wie bereits oben erwähnt, können erfindungsgemäß alle Pflanzen und deren Teile behandelt werden. In einer bevorzugten Ausführungsform werden wild vorkommende oder durch konventionelle biologische Zuchtmethoden, wie Kreuzung oder Protoplastenfusion erhaltenen Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert.

Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

Je nach Pflanzenarten bzw. Pflanzensorten, deren Standort und Wachstumsbedingungen (Böden, Klima, Vegetationsperiode, Ernährung) können durch die erfindungsgemäße Behandlung auch überadditive ("synergistische") Effekte auftreten. So sind beispielsweise erniedrigte Aufwandmengen und/oder Erweiterungen des Wirkungsspektrums und/oder eine Verstärkung der Wirkung der erfindungsgemäß verwendbaren Stoffe und Mittel, besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte möglich, die über die eigentlich zu erwartenden Effekte hinausgehen.

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehr der Pflanzen gegen tierische und mikrobielle Schädlinge, wie gegenüber Insekten, Milben, pflanzenpathogenen Pilzen, Bakterien und/oder Viren sowie eine erhöhte Toleranz der Pflanzen gegen bestimmte herbizide Wirkstoffe. Als Beispiele transgener Pflanzen werden die wichtigen Kulturpflanzen, wie Getreide (Weizen, Reis), Mais, Soja, Kartoffel, Baumwolle, Raps sowie Obstpflanzen (mit den Früchten Äpfel, Birnen, Zitrusfrüchten und Weintrauben) erwähnt, wobei Mais, Soja, Kartoffel, Baumwolle und Raps besonders hervorgehoben werden. Als Eigenschaften ("Traits") werden besonders hervorgehoben die erhöhte Abwehr der Pflanzen gegen Insekten durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden (im folgenden "Bt Pflanzen"). Als Eigenschaften ("Traits") werden auch besonders hervorgehoben die erhöhte Abwehr von Pflanzen gegen Pilze, Bakterien und Viren durch Systemische Akquirierte Resistenz (SAR), Systemin, Phytoalexine, Elicitoren sowie Resistenzgene und entsprechend exprimierte Proteine und Toxine. Als Eigenschaften ("Traits") werden weiterhin besonders hervorgehoben die erhöhte Toleranz der Pflanzen gegenüber bestimmten herbiziden Wirkstoffen, beispielsweise Inüdazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele für "Bt Pflanzen" seien Maissorten, Baumwollsorten, Sojasorten und Kartoffelsorten genannt, die unter den Handelsbezeichnungen YIELD GARD® (z.B. Mais, Baumwolle, Soja), KnockOut® (z.B. Mais), StarLink® (z.B. Mais), Bollgard® (Baumwolle), Nucoton® (Baumwolle) und NewLeaf® (Kartoffel) vertrieben werden. Als Beispiele für Herbizid tolerante Pflanzen seien Maissorten, Baumwollsorten und Sojasorten genannt, die unter den Handelsbezeichnungen Roundup Ready® (Toleranz gegen Glyphosate z.B. Mais, Baumwolle, Soja), Liberty Zink® (Toleranz gegen Phosphinotricin, z.B. Raps), IMI® (Toleranz gegen Imidazolinone) und STS® (Toleranz gegen Sulfonylharnstoffe z.B. Mais) vertrieben werden. Als Herbizid resistente (konventionell auf Herbizid-Toleranz gezüchtete) Pflanzen seien auch die unter der Bezeichnung Clearfield® vertriebenen Sorten (z.B. Mais) erwähnt. Selbstverständlich gelten diese Aussagen auch für in der Zukunft entwickelte bzw. zukünftig auf den Markt kommende Pflanzensorten mit diesen oder zukünftig entwickelten genetischen Eigenschaften ("Traits").

Die aufgeführte Pflanzen können besonders vorteilhaft erfindungsgemäß mit den Verbindungen der allgemeinen Formel (I) bzw. den erfindungsgemäßen Wirkstoffmischungen behandelt werden. Die bei den Wirkstoffen bzw. Mischungen oben angegebenen Vorzugsbereiche gelten auch für die Behandlung dieser Pflanzen. Besonders hervorgehoben sei die Pflanzenbehandlung mit den im vorliegenden Text speziell aufgeführten Verbindungen bzw. Mischungen.

### Herstellungsbeispiel

### Beispiel 1

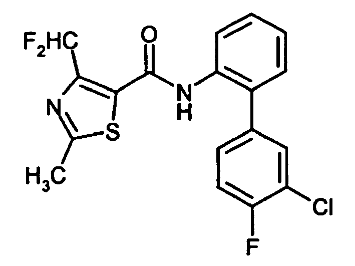

### Verfahren a)

0,288 g (1,3 mmol) 3'-Chlor-4'-fluor-1,1'-biphenyl-2-amin und 0,33 g (1,5 mmol) 2-Methyl-4-(difluormethyl)-1,3-thiazol-5-carbonylchlorid werden in 6 ml Tetrahydrofuran gelöst und mit 0,36 ml (2,6 mmol) Triethylamin versetzt. Die Reaktionslösung wird für 16 h bei 60°C gerührt. Zur Aufarbeitung wird aufkonzentriert und mit Cyclohexän/Essigsäureethylester an Kieselgel chromatographiert.

Man erhält 0,434 g (84% d. Th.) an *N*-(3'-Chlor-4'-fluor-1,1'-biphenyl-2-yl)-2-methyl-4-(difluormethyl)-1,3-thiazol-5-carboxamid mit dem logP (pH2,3) = 3,28.

Analog Beispiel 1, sowie entsprechend den Angaben in den allgemeinen Beschreibungen der Verfahren a) und b), werden die in der nachstehenden Tabelle 1 genannten Verbindungen der Formel (I) erhalten.

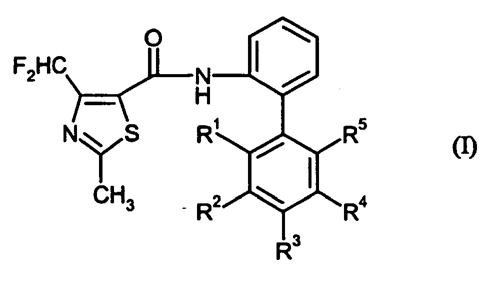

**Tabelle 1**

| **Bsp.** | **R¹** | **R²** | **R³** | **R⁴** | **R⁵** | **logP** |
|---|---|---|---|---|---|---|
| 2 | H | H | Br | H | H | 3,47 |
| 3 | H | H | CF₃ | H | H | 3,52 |
| 4 | H | Cl | H | H | H | 3,37 |
| 5 | H | H | OCF₃ | H | H | 3,72 |
| 6 | H | H | SCH₃ | H | H | 3,39 |
| 7 | H | H | F | H | H | 3,09 |
| 8 | H | Cl | Cl | H | H | 3,58 |
| 9 | Cl | H | Cl | H | H | 3,58 |
| 10 | CH₃ | H | Cl | H | H | 3,77 |
| 11 | H | F | Cl | H | H | 3,29 |
| 12 | H | Cl | CH₃ | H | H | 3,62 |
| 13 | F | H | Cl | H | H | 3,26 |
| 14 | H | F | H | Cl | H | 3,36 |
| 15 | F | H | Br | H | H | 3,34 |
| 16 | H | CH₃ | Cl | H | H | 3,66 |
| 17 | H | Cl | H | Cl | H | 3,7 |
| 18 | H | F | H | F | H | 3,07 |
| 19 | H | CF₃ | Cl | H | H | 3,66 |
| 20 | H | F | F | H | H | 3,04 |
| 21 | H | H | Cl | H | H | 3,27 |
| 22 | H | F | Br | H | H | 3,36 |
| 23 | H | F | CF₃ | H | H | 3,43 |
| 24 | F | H | F | H | H | 2,93 |
| 25 | H | H | CN | H | H | 2,51 |
| 26 | H | H | H | H | H | 3,50 |

### Herstellung eines Vorproduktes der Formel (III)

### Beispiel (III-1)

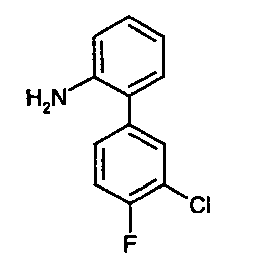

38,8 g (223 mmol) 3-Chlor-4-fluorphenylboronsäure, 40,6 g (186 mmol) 2-Iodanilin werden in 220 ml Toluol, 22 ml Ethanol und 45 ml einer 4 M Natriumhydrogencarbonatlösung unter Argon gelöst. Hierzu gibt man 4,3 g (4 mmol) Tetrakis(triphenylphosphin)palladium(0) lässt die Reaktionslösung 16 Stunden bei 80 °C unter Argon rühren. Die organische Phase wird abgetrennt, über Magnesiumsulfat getrocknet und aufkonzentriert. Der Rückstand wird mit Cyclohexan/Essigsäureethylester an Kieselgel chromatographiert.

Man erhält 22,5 g (48% d. Th.) 3'-Chlor-4'-fluor-1,1'-biphenyl-2-amin (Reinheit 88%) mit dem logP (pH2,3) = 3,01.

Die Bestimmung der in den voranstehenden Tabellen und Herstellungsbeispielen angegebenen logP-Werte erfolgt gemäß EEC-Directive 79/831 Annex V.A8 durch HPLC (High Performance Liquid Chromatography) an einer Phasenumkehrsäule (C 18). Temperatur: 43°C.

Die Bestimmung erfolgt im sauren Bereich bei pH 2.3 mit 0,1 % wässriger Phosphorsäure und Acetonitril als Eluenten; linearer Gradient von 10 % Acetonitril bis 90 % Acetonitril.

Die Eichung erfolgt mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinanderfolgenden Alkanonen).

Die lambda-max-Werte wurden an Hand der UV-Spektren von 200 nm bis 400 nm in den Maxima der chromatographischen Signale ermittelt.

### Anwendungsbeispieie

### Beispiel A

### Venturia - Test (Apfel) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden die Pflanzen mit einer wässrigen Konidiensuspension des Apfelschorferregers ***Venturia inaequalis*** inokuliert und verbleiben dann 1 Tag bei ca. 20°C und 100 % relativer Luftfeuchtigkeit in einer Inkubationskabine.

Die Pflanzen werden dann im Gewächshaus bei ca. 21°C und einer relativen Luftfeuchtigkeit von ca. 90% aufgestellt.

10 Tage nach der Inokulation erfolgt die Auswertung. Dabei bedeutet 0 % ein Wirkungsgrad, der demjenigen der Kontrolle entspricht, während ein Wirkungsgrad von 100 % bedeutet, dass kein Befall beobachtet wird.

| **Tabelle A: Venturia - Test (Apfel) / protektiv** | | | |
|---|---|---|---|
| Bsp. | Wirkstoff | Aufwandmenge an Wirkstoff in g/ha | % Wirkungsgrad |
| Gemäß EP-A 0 545 099: | | | |
| 3.37 | 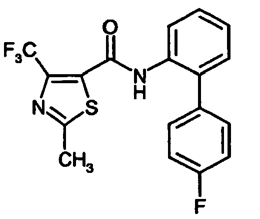 | 10 | 32 |
| Erfindungsgemäß: | | | |
| 2 | 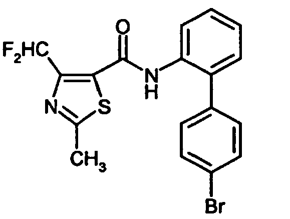 | 10 | 100 |
| 3 | 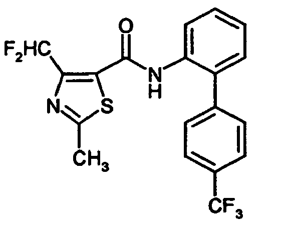 | 10 | 100 |
| 4 | 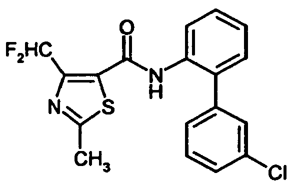 | 10 | 100 |
| 5 | 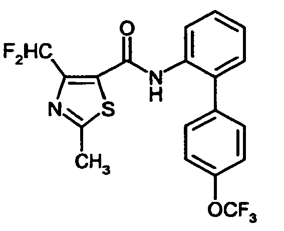 | 10 | 87 |
| 6 | 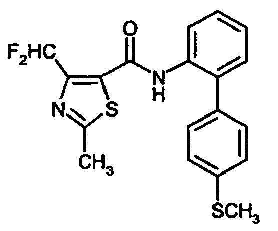 | 10 | 99 |
| 7 | 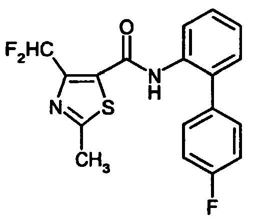 | 10 | 100 |
| 9 | 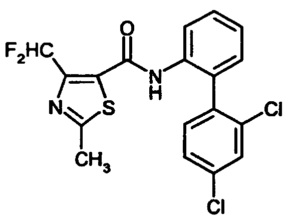 | 10 | 98 |
| 10 | 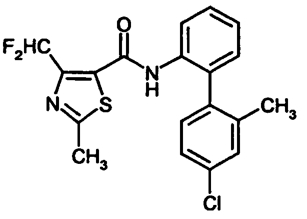 | 10 | 100 |
| 11 | 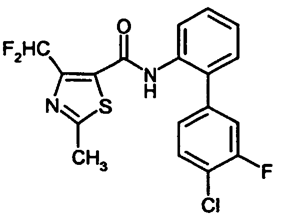 | 10 | 100 |
| 12 | 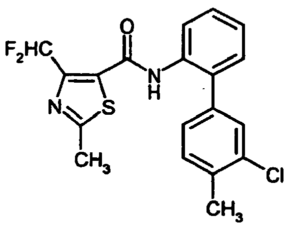 | 10 | 100 |

### Beispiel B

### Botrytis - Test (Bohne) / protektiv

| | |
|---|---|
| Lösungsmittel: | 24,5 Gewichtsteile Aceton |
| | 24,5 Gewichtsteile Dimethylacetamid |
| Emulgator: | 1,0 Gewichtsteile Alkyl-Aryl-Polyglykolether |

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit werden junge Pflanzen mit der Wirkstoffzubereitung in der angegebenen Aufwandmenge besprüht. Nach Antrocknen des Spritzbelages werden auf jedes Blatt 2 kleine mit ***Botrytis cinerea*** bewachsene Agarstückchen aufgelegt. Die inokulierten Pflanzen werden in einer abgedunkelten Kammer bei ca. 20°C und 100 % relativer Luftfeuchtigkeit aufgestellt.

2 Tage nach der Inokulation wird die Größe der Befallsflecken auf den Blättern ausgewertet. Dabei bedeutet 0 % ein Wirkungsgrad, der demjenigen der Kontrolle entspricht, während ein Wirkungsgrad von 100 % bedeutet, dass kein Befall beobachtet wird.

| **Tabelle B:** | | | |
|---|---|---|---|
| **Botrytis - Test (Bohne) / protektiv** | | | |
| Bsp. | Wirkstoff | Aufwandmenge an Wirkstoff in g/ha | % Wirkungsgrad |
| Gemäß EP-A 0 545 099: | | | |
| 3.37 | 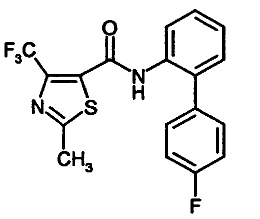 | 100 | 50 |
| Erfindungsgemäß: | | | |
| 2 | 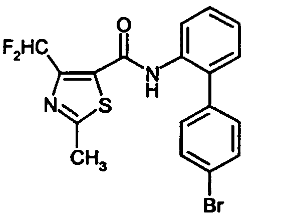 | 100 | 100 |
| 3 | 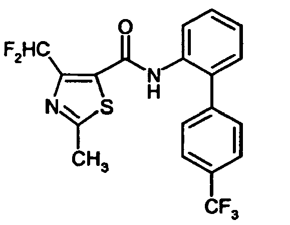 | 100 | 100 |
| 4 | 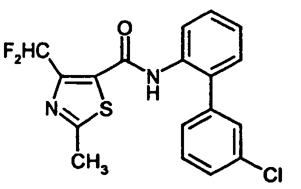 | 100 | 100 |
| 5 | 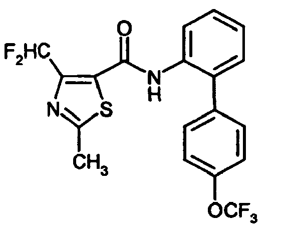 | 100 | 79 |
| 6 | 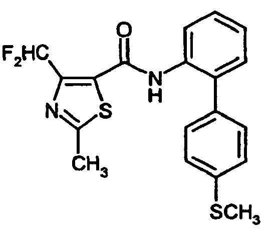 | 100 | 94 |
| 7 | 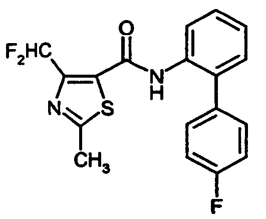 | 100 | 94 |
| 9 | 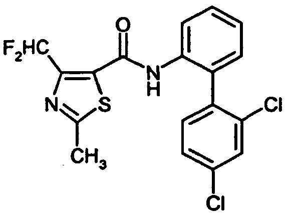 | 100 | 95 |
| 10 | 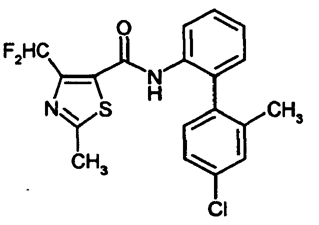 | 100 | 100 |
| 11 | 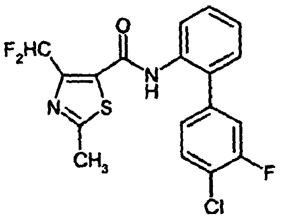 | 100 | 100 |
| 12 | 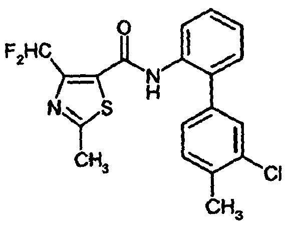 | 100 | 100 |

### Beispiel C

### In vitro-Test zur ED₅₀-Bestimmung bei Mikroorganismen

In die Kavitäten von Mikrotiterplatten wird eine methanolische Lösung des zu prüfenden Wirkstoffs, versetzt mit dem Emulgator PS 16, pipettiert. Nachdem das Lösungsmittel abgedampft ist, werden je Kavität 200 µl Potato-Dextrose-Medium hinzugefügt.

Das Medium wurde vorher mit einer geeigneten Konzentration von Sporen bzw. Mycel des zu prüfenden Pilzes versetzt.

Die resultierenden Konzentrationen des Wirkstoffs betragen 0.1, 1, 10 und 100 ppm. Die resultierende Konzentration des Emulgators beträgt 300 ppm.

Die Platten werden anschließend 3-5 Tage auf einem Schüttler bei einer Temperatur von 22°C inkubiert bis in der unbehandelten Kontrolle ein ausreichendes Wachstum feststellbar ist.

Die Auswertung erfolgt photometrisch bei einer Wellenlänge von 620 nm. Aus den Messdaten der verschiedenen Konzentrationen wird die Wirkstoffdosis, die zu einer 50%igen Hemmung des Pilzwachstums gegenüber der unbehandelten Kontrolle führt (ED₅₀), berechnet.

| **Tabelle C:** | | | |
|---|---|---|---|
| **In vitro-Test zur ED₅₀-Bestimmung bei Mikroorganismen** | | | |
| Bsp. | Wirkstoff | Mikroorganismus | ED₅₀-Wert |
| Gemäß EP-A 0 545 099: | | | |
| 3.37 | 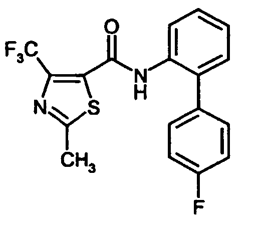 | Rhizoctonia solani Septoria tritici | > 100 84,24 |
| Erfindungsgemäß: | | | |
| 3 | 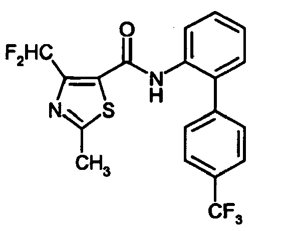 | Rhizoctonia solani Septoria tritici | < 0,1 0,36 |
| 4 | 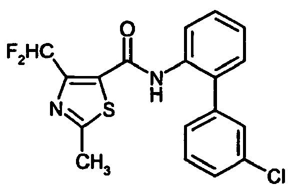 | Rhizoctonia solani Septoria tritici | < 0,1 0,45 |
| 5 | 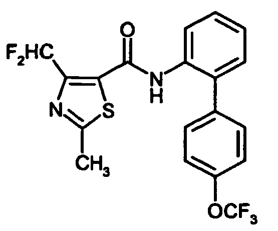 | Rhizoctonia solani Septoria tritici | < 0,1 0,25 |
| 6 | 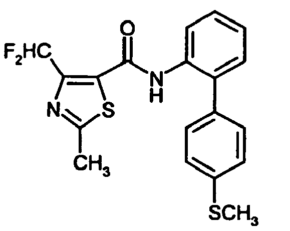 | Rhizoctonia solani Septoria tritici | 3,25 3,51 |
| 7 | 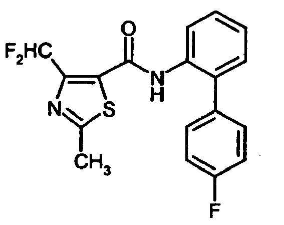 | Rhizoctonia solani Septoria tritici | 0,48 0,68 |

## Patentansprüche

1. Difluormethylthiazolylcarboxanilide der Formel (I) 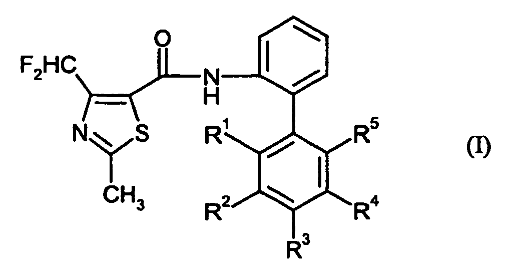 in welcher
R¹, R², R³, R⁴ und R⁵ unabhängig voneinander für Wasserstoff, Halogen, Cyano, Nitro, C₁-C₆-Alkyl, C₂-C₆-Alkenyl, C₁-C₄-Alkoxy, C₁-C₄- Alkylthio, C₁-C₄-Alkylsulfonyl, C₃-C₆-Cycloalkyl, oder für C₁-C₄- Halogenalkyl, C₁-C₄-Halogenalkoxy, C₁-C₄-Halogenalkylthio oder C₁- C₄-Halogenalkylsulfonyl mit jeweils 1 bis 5 Halogenatomen stehen, mit der Maßgabe, dass R¹, R², R³, R⁴ und R⁵ nicht gleichzeitig für Wasserstoff stehen,
R¹ und R² oder R² und R³ außerdem gemeinsam für gegebenenfalls durch Halogen oder C₁-C₆-Alkyl substituiertes Alkenylen stehen.

2. Difluormethylthiazolylcarboxanilide der Formel (I) gemäß Anspruch 1, in welcher
R¹, R², R³, R⁴ und R⁵ unabhängig voneinander für Wasserstoff, Fluor, Chlor, Brom, Cyano, Nitro, Methyl, Ethyl, n- oder i-Propyl, n-, i-, s- oder t- Butyl, Methoxy, Ethoxy, Methylthio, Ethylthio, n- oder i-Propylthio, Cyclopropyl, Trifluormethyl, Trichlormethyl, Trifluorethyl, Difluor- methoxy, Trifluormethoxy, Difluorchlormethoxy, Trifluorethoxy, Difluormethylthio, Difluorchlormethylthio oder Trifluormethylthio stehen, mit der Maßgabe, dass R¹, R², R³, R⁴ und R⁵ nicht gleichzeitig für Wasserstoff stehen,
R¹ und R² oder R² und R³ außerdem gemeinsam für gegebenenfalls durch Fluor, Chlor, Brom oder Methyl substituiertes Butadienylen stehen.

3. Difluormethylthiazolylcarboxanilide der Formel (I) gemäß Anspruch 1, in welcher
R¹, R², R³, R⁴ und R⁵ unabhängig voneinander für Wasserstoff, Fluor, Chlor, Brom, Methyl, Methoxy, Methylthio, Trifluormethyl, Difluormethoxy, Trifluormethoxy, Difluormethylthio oder Trifluormethylthio stehen, mit der Maßgabe, dass R¹, R², R³, R⁴ und R⁵ nicht gleichzeitig für Wasserstoff stehen.

4. Difluormethylthiazolylcarboxanilide der Formel (I) gemäß Anspruch 1, in welcher
R¹, R², R⁴ und R⁵ jeweils für Wasserstoff stehen und
R³ die in Anspruch 1 angegebenen Bedeutungen hat.

5. Difluormethylthiazolylcarboxanilide der Formel (I) gemäß Anspruch 1, in welcher
R¹, R², R⁴ und R⁵ jeweils für Wasserstoff stehen und
R³ für Fluor, Chlor, Brom, Methyl, Trifluormethyl, Trifluormethoxy oder Trifluormethylthio steht.

6. Difluormethylthiazolylcarboxanilide der Formel (I) gemäß Anspruch 1, in welcher
R², R⁴ und R⁵ für jeweils Wasserstoff stehen und
R¹ und R³ unabhängig voneinander die in Anspruch 1 angegebenen Bedeu- tungen haben.

7. Difluormethylthiazolylcarboxanilide der Formel (I) gemäß Anspruch 1, in welcher
R², R⁴ und R⁵ für jeweils Wasserstoff stehen und
R¹ und R³ unabhängig voneinander für Fluor, Chlor, Brom, Methyl oder Trifluormethyl stehen.

8. Difluormethylthiazolylcarboxanilide der Formel (I) gemäß Anspruch 1, in welcher
R¹, R⁴ und R⁵ jeweils für Wasserstoff stehen und
R² und R³ unabhängig voneinander die in Anspruch 1 angegebenen Bedeu- tungen haben.

9. Difluormethylthiazolylcarboxanilide der Formel (I) gemäß Anspruch 1, in welcher
R¹, R⁴ und R⁵ jeweils für Wasserstoff stehen und
R² und R³ unabhängig voneinander für Fluor, Chlor, Brom, Methyl oder Trifluormethyl stehen.

10. Difluormethylthiazolylcarboxanilide der Formel (I) gemäß Anspruch 1, in welcher
R¹, R³ und R⁵ jeweils für Wasserstoff stehen und
R² und R⁴ unabhängig voneinander die in Anspruch 1 angegebenen Bedeu- haben.

11. Difluormethylthiazolylcarboxanilide der Formel (I) gemäß Anspruch 1, in welcher
R¹, R³ und R⁵ jeweils für Wasserstoff stehen und
R² und R⁴ unabhängig voneinander für Fluor, Chlor, Brom, Methyl oder Trifluormethyl stehen.

12. Difluormethylthiazolylcarboxanilide der Formel (I) gemäß Anspruch 1, in welcher R⁵ für Wasserstoff steht.

13. Verfahren zum Herstellen von Difluormethylthiazolylcarboxaniliden der Formel (I) gemäß Anspruch 1, **dadurch gekennzeichnet, dass** man
a) Difluormethylthiazolylcarbonsäurehalogenide der Formel (II) 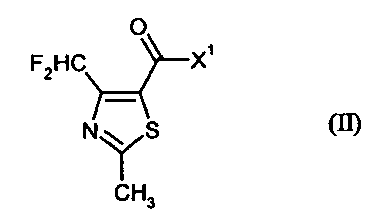 in welcher
x¹ für Halogen steht,
mit Anilinderivaten der Formel (III) 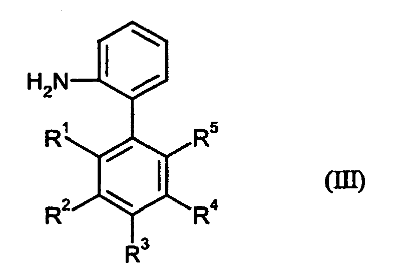 in welcher
R¹, R², R³, R⁴ und R⁵ die in Anspruch 1 angegebenen Bedeutungen haben,
gegebenenfalls in Gegenwart eines Säurebindemittels und gegebenenfalls in Gegenwart eines Verdünnungsmittels umsetzt, oder
b) Difluormethylthiazolylcarboxhalogenanilide der Formel (IV) 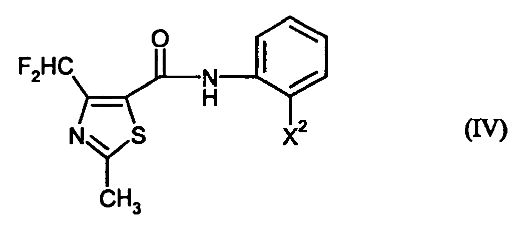 in welcher
X² für Brom oder Iod steht,
mit Boronsäurederivaten der Formel (V) 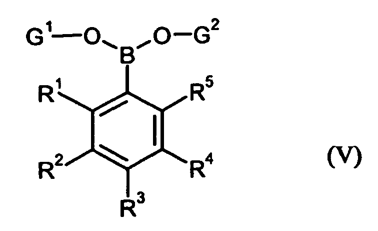 in welcher
R¹, R², R³, R⁴ und R⁵ die in Anspruch 1 angegebenen Bedeutungen haben,
G¹ und G² jeweils für Wasserstoff oder zusammen für Tetramethyl- ethylen stehen,
in Gegenwart eines Katalysators, gegebenenfalls in Gegenwart eines Säurebindemittels und gegebenenfalls in Gegenwart eines Verdünnungsmittels umsetzt, oder
c) Difluormethylthiazolylcarboxanilid-Boronsäure-Derivate der Formel (VI) 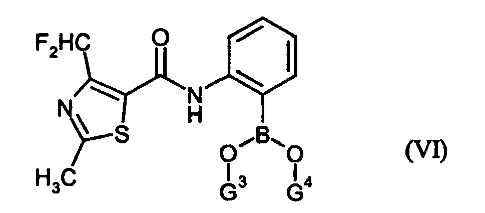 in welcher
G³ und G⁴ jeweils für Wasserstoff oder zusammen für Tetramethyl- ethylen stehen,
mit Halogenbenzolderivaten der Formel (VII) 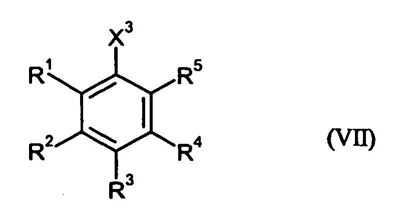 in welcher
R¹, R², R³, R⁴ und R⁵ die in Anspruch 1 angegebenen Bedeutungen haben,
X³ für Brom, Iod oder Trifluormethylsulfonyloxy steht,
in Gegenwart eines Katalysators, gegebenenfalls in Gegenwart eines Säurebindemittels und gegebenenfalls in Gegenwart eines Verdünnungsmittels umsetzt.

14. Mittel zum Bekämpfen unerwünschter Mikroorganismen, **gekennzeichnet durch** einen Gehalt an mindestens einem Thiazolylcarboxanilid der Formel (I) gemäß Anspruch 1 neben Streckmitteln und/oder oberflächenaktiven Stoffen.

15. Verwendung von Thiazolylcarboxaniliden der Formel (I) gemäß Anspruch 1 zum Bekämpfen unerwünschter Mikroorganismen.

16. Verfahren zum Bekämpfen unerwünschter Mikroorganismen, **dadurch gekennzeichnet, dass** man Thiazolylcarboxanilide der Formel (I) gemäß Anspruch 1 auf die Mikroorganismen und/oder deren Lebensraum ausbringt.

17. Verfahren zum Herstellen von Mitteln zum Bekämpfen unerwünschter Mikroorganismen, **dadurch gekennzeichnet, dass** man Thiazolylcarboxanilide der Formel (I) gemäß Anspruch 1 mit Streckmitteln und/oder oberflächenaktiven Stoffen vermischt.

18. Difluormethylthiazolylcarboxhalogenanilide der Formel (IV) 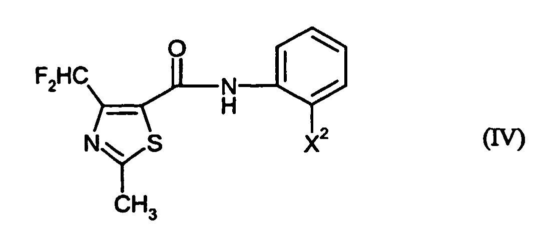 in welcher
X² für Brom oder Iod steht.

19. Verfahren zum Herstellen von Difluormethylthiazolylcarboxhalogenaniliden der Formel (IV) gemäß Anspruch 18, **dadurch gekennzeichnet, dass** man
d) Difluormethylthiazolylcarbonsäurehalogenide der Formel (II) 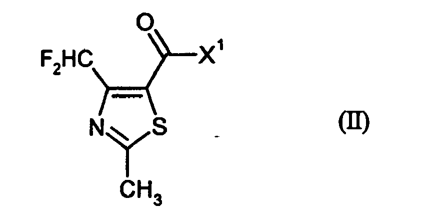 in welcher
X¹ für Halogen steht,
mit 2-Bromanilin oder 2-Iodanilin umsetzt.

20. Difluormethylthiazolylcarboxanilid-Boronsäure-Derivate der Formel (VI) 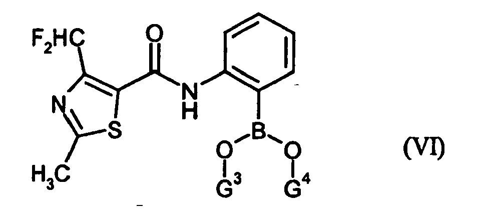 in welcher
G³ und G⁴ jeweils für Wasserstoff oder zusammen für Tetramethylethylen stehen.

21. Verfahren zum Herstellen von Difluormethylthiazolylcarboxanilid-Boronsäure-Derivate der Formel (VI) gemäß Anspruch 20, **dadurch gekennzeichnet, dass** man
e) Difluormethylthiazolylcarbonsäurehalogenide der Formel (II) 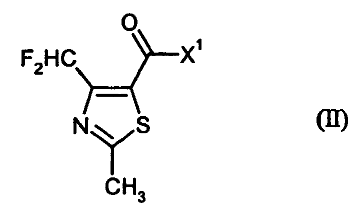 in welcher
X¹ für Halogen steht,
mit Anilinboronsäurederivaten der Formel (VIII) 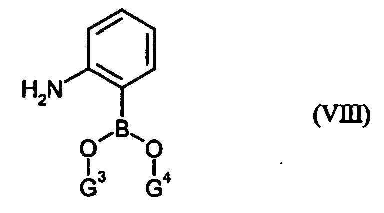 in welcher
G³ und G⁴ die in Anspruch 20 angegebenen Bedeutungen haben,
gegebenenfalls in Gegenwart eines Säurebindemittels und gegebenenfalls in Gegenwart eines Verdünnungsmittels umsetzt.

## Claims

1. Difluoromethylthiazolylcarboxanilides of the formula (I) 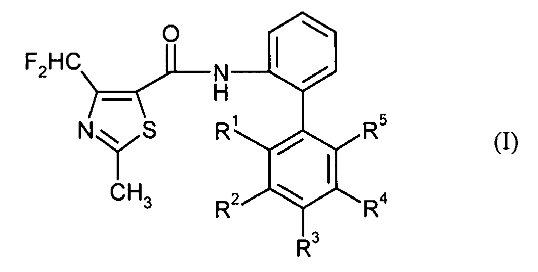 in which
R¹, R², R³, R⁴ and R⁵ independently of one another represent hydrogen, halogen, cyano, nitro, C₁-C₆-alkyl, C₂-C₆-alkenyl, C₁-C₄-alkoxy, C₁-C₄- alkylthio, C₁-C₄-alkylsulphonyl, C₃-C₆-cycloalkyl, or represent C₁-C₄- haloalkyl, C₁-C₄-haloalkoxy, C₁-C₄-haloalkylthio or C₁-C₄- haloalkylsulphonyl having in each case 1 to 5 halogen atoms, with the proviso that R¹, R², R³, R⁴ and R⁵ do not simultaneously represent hydrogen,
R¹ and R² or R² and R³ furthermore together represent optionally halogen- or C₁-C₆-alkyl-substituted alkenylene.

2. Difluoromethylthiazolylcarboxanilides of the formula (I) according to Claim 1, in which
R¹, R², R³, R⁴ and R⁵ independently of one another represent hydrogen, fluorine, chlorine, bromine, cyano, nitro, methyl, ethyl, n- or i-propyl, n- , i-, s- or t-butyl, methoxy, ethoxy, methylthio, ethylthio, n- or i- propylthio, cyclopropyl, trifluoromethyl, trichloromethyl, trifluoroethyl, difluoromethoxy, trifluoromethoxy, difluorochloromethoxy, trifluoroethoxy, difluoromethylthio, difluorochloromethylthio or trifluoromethylthio, with the proviso that R¹, R², R³, R⁴ and R⁵ do not simultaneously represent hydrogen,
R¹ and R² or R² and R³ furthermore together represent optionally fluorine-, chlorine-, bromine- or methyl-substituted butadienylene.

3. Difluoromethylthiazolylcarboxanilides of the formula (I) according to Claim 1, in which
R¹, R², R³, R⁴ and R⁵ independently of one another represent hydrogen, fluorine, chlorine, bromine, methyl, methoxy, methylthio, trifluoro- methyl, difluoromethoxy, trifluoromethoxy, difluoromethylthio or trifluoromethylthio, with the proviso that R¹, R², R³, R⁴ and R⁵ do not simultaneously represent hydrogen.

4. Difluoromethylthiazolylcarboxanilides of the formula (I) according to Claim 1, in which
R¹, R², R⁴ and R⁵ each represent hydrogen and
R³ is as defined in Claim 1.

5. Difluoromethylthiazolylcarboxanilides of the formula (I) according to Claim 1, in which
R¹, R², R⁴ and R⁵ each represent hydrogen and
R³ represents fluorine, chlorine, bromine, methyl, trifluoromethyl, trifluoromethoxy or trifluoromethylthio.

6. Difluoromethylthiazolylcarboxanilides of the formula (I) according to Claim 1, in which
R², R⁴ and R⁵ each represent hydrogen and
R¹ and R³ independently of one another are as defined in Claim 1.

7. Difluoromethylthiazolylcarboxanilides of the formula (I) according to Claim 1, in which
R², R⁴ and R⁵ each represent hydrogen and
R¹ and R³ independently of one another represent fluorine, chlorine, bromine, methyl or trifluoromethyl.

8. Difluoromethylthiazolylcarboxanilides of the formula (I) according to Claim 1, in which
R¹, R⁴ and R⁵ each represent hydrogen and
R² and R³ independently of one another are as defined in Claim 1.

9. Difluoromethylthiazolylcarboxanilides of the formula (I) according to Claim 1, in which
R¹, R⁴ and R⁵ each represent hydrogen and
R² and R³ independently of one another represent fluorine, chlorine, bromine, methyl or trifluoromethyl.

10. Difluoromethylthiazolylcarboxanilides of the formula (I) according to Claim 1, in which
R¹, R³ and R⁵ each represent hydrogen and
R² and R⁴ independently of one another are as defined in Claim 1.

11. Difluoromethylthiazolylcarboxanilides of the formula (I) according to Claim 1, in which
R¹, R³ and R⁵ each represent hydrogen and
R² and R⁴ independently of one another represent fluorine, chlorine, bromine, methyl or trifluoromethyl.

12. Difluoromethylthiazolylcarboxanilides of the formula (I) according to Claim 1, in which R⁵ represents hydrogen.

13. Process for preparing difluoromethylthiazolylcarboxanilides of the formula (I) according to Claim 1, **characterized in that**
a) difluoromethylthiazolylcarbonyl halides of the formula (II) 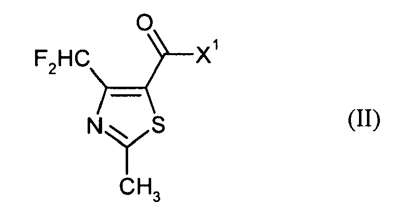 in which
X¹ represents halogen,
are reacted with aniline derivatives of the formula (III) 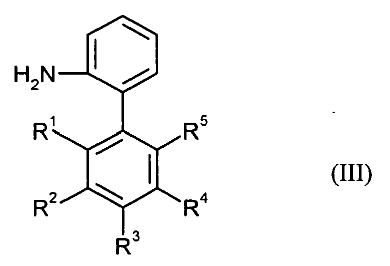 in which
R¹, R², R³, R⁴ and R⁵ are as defined in Claim 1,
if appropriate in the presence of an acid binder and if appropriate in the presence of a diluent, or
b) difluoromethylthiazolylcarboxhaloanilides of the formula (IV) 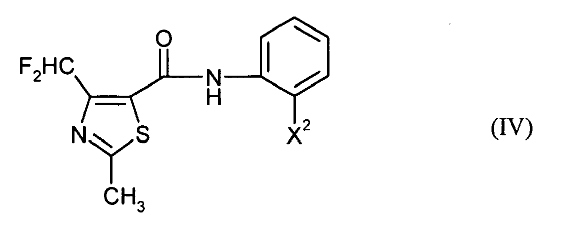 in which
X² represents bromine or iodine,
are reacted with boronic acid derivatives of the formula (V) 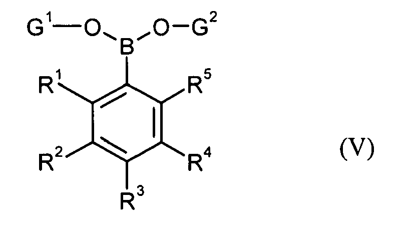 in which
R¹, R², R³, R⁴ and R⁵ are as defined in Claim 1,
G¹ and G² each represent hydrogen or together represent tetramethyl- ethylene,
in the presence of a catalyst, if appropriate in the presence of an acid binder and if appropriate in the presence of a diluent, or
c) difluoromethylthiazolylcarboxanilide boronic acid derivatives of the formula (VI) 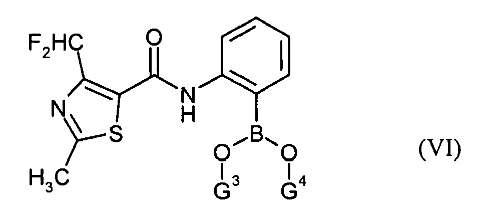 in which
G³ and G⁴ each represent hydrogen or together represent tetramethylethylene,
are reacted with halobenzene derivatives of the formula (VII) 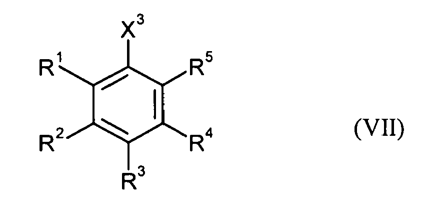 in which
R¹, R², R³, R⁴ and R⁵ are as defined in Claim 1,
X³ represents bromine, iodine or trifluoromethylsulphonyloxy,
in the presence of a catalyst, if appropriate in the presence of an acid binder and if appropriate in the presence of a diluent.

14. Compositions for controlling unwanted microorganisms, **characterized in that** they comprise at least one thiazolylcarboxanilide of the formula (I) according to Claim 1, in addition to extenders and/or surfactants.

15. Use of thiazolylcarboxanilides of the formula (I) according to Claim 1 for controlling unwanted microorganisms.

16. Method for controlling unwanted microorganisms, **characterized in that** thiazolylcarboxanilides of the formula (I) according to Claim 1 are applied to the microorganisms and/or their habitat.

17. Process for preparing compositions for controlling unwanted microorganisms, **characterized in that** thiazolylcarboxanilides of the formula (I) according to Claim 1 are mixed with extenders and/or surfactants.

18. Difluoromethylthiazolylcarboxhaloanilides of the formula (IV) 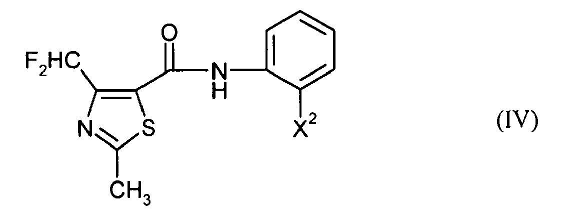 in which
X² represents bromine or iodine.

19. Process for preparing difluoromethylthiazolylcarboxhaloanilides of the formula (IV) according to Claim 18, **characterized in that**
d) difluoromethylthiazolylcarbonyl halides of the formula (II) 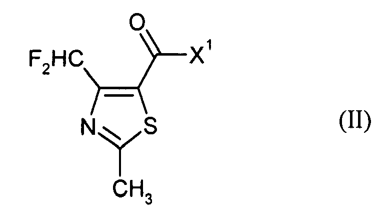 in which
X¹ represents halogen,
are reacted with 2-bromoaniline or 2-iodoaniline.

20. Difluoromethylthiazolylcarboxanilide boronic acid derivatives of the formula (VI) 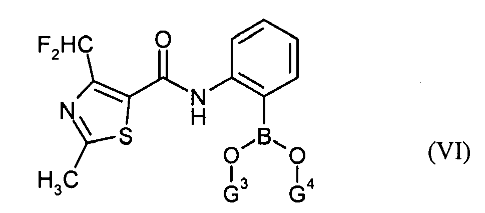 in which
G³ and G⁴ each represent hydrogen or together represent tetramethylethylene.

21. Process for preparing difluoromethylthiazolylcarboxanilide boronic acid derivatives of the formula (VI) according to Claim 20, **characterized in that**
e) difluoromethylthiazolylcarbonyl halides of the formula (II) 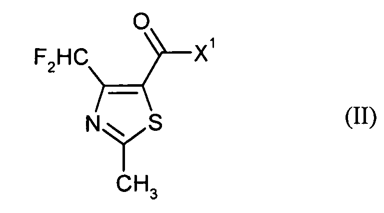 in which
X¹ represents halogen,
are reacted with anilineboronic acid derivatives of the formula (VIII) 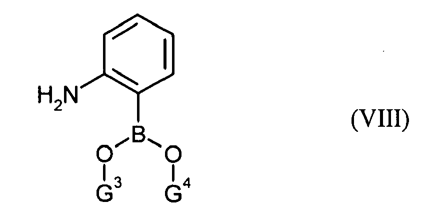 in which
G³ and G⁴ are as defined in Claim 20,
if appropriate in the presence of an acid binder and if appropriate in the presence of a diluent.

## Revendications

1. Difluorométhylthiazolylcarboxanilides de la formule (I) 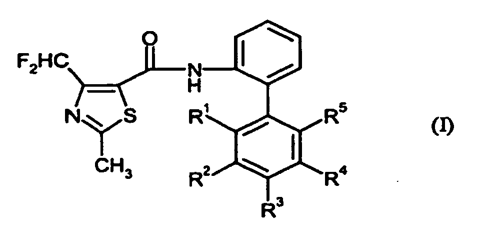 dans laquelle
R¹, R², R³, R⁴ et R⁵ représentent indépendamment l'hydrogène, un atome d'halogène, un groupe cyano, nitro, alkyle en C₁-C₆, alcényle en C₂-C₆, alcoxy en C₁-C₄, alkylthio en C₁-C₄, alkylsulfonyle en C₁-C₄, cycloalkyle en C₃-C₆ ou halogénoalkyle en C₁-C₄, halogénoalcoxy en C₁-C₄, halogénoalkylthio en C₁-C₄ ou halogénoalkylsulfonyle en C₁-C₄ avec à chaque fois de 1 à 5 atomes d'halogène, à condition que R¹, R², R³, R⁴ et R⁵ ne soient pas simultanément l'hydrogène,
R¹ et R², ou R² et R³ représentent de plus ensemble un groupe alcénylène éventuellement substitué par un atome d'halogène ou un groupe alkyle en C₁-C₆.

2. Difluorométhylthiazolylcarboxanilides de la formule (I) selon la revendication 1, dans lesquels
R¹, R², R³, R⁴ et R⁵ représentent indépendamment l'hydrogène, le fluor, le chlore, le brome, un groupe cyano, nitro, méthyle, éthyle, n- ou i-propyle, n-, i-, s- ou t-butyle, méthoxy, éthoxy, méthylthio, éthylthio, n-ou i-propylthio, cyclopropyle, trifluorométhyle, trichlorométhyle, trifluoroéthyle, difluorométhoxy, trifluorométhoxy, difluorochlorométhoxy, trifluoroéthoxy, difluorométhylthio, difluorochlorométhylthio ou trifluorométhylthio, à condition que R¹, R², R³, R⁴ et R⁵ ne soient pas simultanément l'hydrogène,
R¹ et R², ou R² et R³ représentent de plus un groupe butadiénylène éventuellement substitué par le fluor, le chlore, le brome ou un groupe méthyle.

3. Difluorométhylthiazolylcarboxanilides de la formule (I) selon la revendication 1, dans lesquels
R¹, R², R³, R⁴ et R⁵ représentent indépendamment l'hydrogène, le fluor, le chlore, le brome, un groupe méthyle, méthoxy, méthylthio, trifluorométhyle, difluorométhoxy, trifluorométhoxy, difluorométhylthio ou trifluorométhylthio, à condition que R¹, R², R³, R⁴ et R⁵ ne soient pas simultanément l'hydrogène.

4. Difluorométhylthiazolylcarboxanilides de la formule (I) selon la revendication 1, dans lesquels
R¹, R², R⁴ et R⁵ sont chacun l'hydrogène et
R³ a la signification indiquée dans la revendication 1.

5. Difluorométhylthiazolylcarboxanilides de la formule (I) selon la revendication 1, dans lesquels
R¹, R², R⁴ et R⁵ sont chacun l'hydrogène et
R³ représente le fluor, le chlore, le brome, un groupe méthyle, trifluorométhyle, trifluorométhoxy ou trifluorométhylthio.

6. Difluorométhylthiazolylcarboxanilides de la formule (I) selon la revendication 1, dans lesquels
R², R⁴ et R⁵ sont chacun l'hydrogène et
R¹ et R³ ont indépendamment l'un de l'autre les significations indiquées dans la revendication 1.

7. Difluorométhylthiazolylcarboxanilides de la formule (I) selon la revendication 1, dans lesquels
R², R⁴ et R⁵ sont chacun l'hydrogène et
R¹ et R³ représentent indépendamment l'un de l'autre le fluor, le chlore, le brome, un groupe méthyle ou trifluorométhyle.

8. Difluorométhylthiazolylcarboxanilides de la formule (I) selon la revendication 1, dans lesquels
R¹, R⁴ et R⁵ sont chacun l'hydrogène et
R² et R³ ont indépendamment l'un de l'autre les significations indiquées dans la revendication 1.

9. Difluorométhylthiazolylcarboxanilides de la formule (I) selon la revendication 1, dans lesquels
R¹, R⁴ et R⁵ sont chacun l'hydrogène et
R² et R³ représentent indépendamment l'un de l'autre le fluor, le chlore, le brome, un groupe méthyle ou trifluorométhyle.

10. Difluorométhylthiazolylcarboxanilides de la formule (I) selon la revendication 1, dans lesquels
R¹, R³ et R⁵ sont chacun l'hydrogène et
R² et R⁴ ont indépendamment l'un de l'autre les significations indiquées dans la revendication 1.

11. Difluorométhylthiazolylcarboxanilides de la formule (I) selon la revendication 1, dans lesquels
R¹, R³ et R⁵ sont chacun l'hydrogène et
R² et R⁴ représentent indépendamment l'un de l'autre le fluor, le chlore, le brome, un groupe méthyle ou trifluorométhyle.

12. Difluorométhylthiazolylcarboxanilides de la formule (I) selon la revendication 1, dans lesquels R⁵ représente l'hydrogène.

13. Procédé pour la préparation de difluorométhylthiazolylcarboxanilides de la formule (I) selon la revendication 1, **caractérisé en ce que** l'on fait réagir
a) des halogénures d'acides difluorométhylthiazolylcarboxyliques de la formule (II) 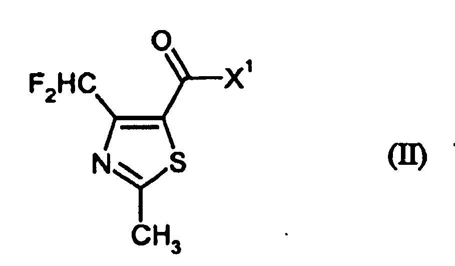 dans laquelle
X¹ représente un atome d'halogène,
avec des dérivés d'anilines de la formule (III) 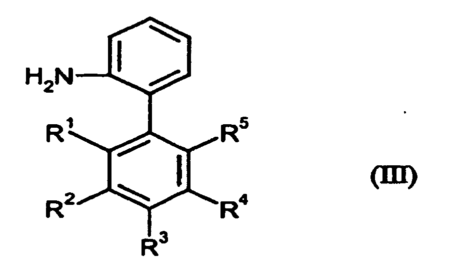 dans laquelle
R¹, R², R³ R⁴ et R⁵ ont les significations indiquées dans la revendication 1,
éventuellement en présence d'un liant acide et éventuellement en présence d'un agent de dilution, ou
b) des difluorométhylthiazolylcarboxyhalogénoanilides de la formule (IV) 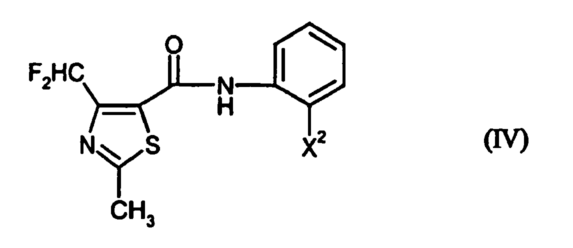 dans laquelle
X² représente le brome ou l'iode,
avec des dérivés d'acides boriques de la formule (V) 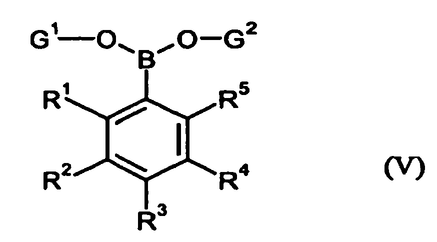 dans laquelle
R¹, R², R³, R⁴ et R⁵ ont les significations indiquées dans la revendication 1,
G¹ et G² représentent chacun l'hydrogène ou ensemble un groupe tétraméthyléthylène,
en présence d'un catalyseur, éventuellement en présence d'un liant acide et éventuellement en présence d'un agent de dilution, ou
c) des dérivés de difluorométhylthiazolylcarboxanilides-acides boriques de la formule (VI) 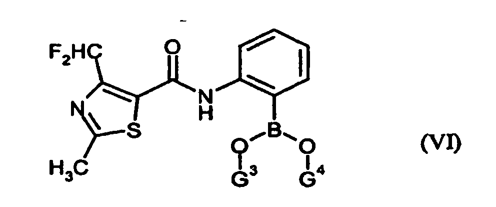 dans laquelle
G³ et G⁴ sont chacun l'hydrogène ou représentent ensemble un groupe tétraméthyléthylène,
avec des dérivés d'halogénobenzènes de la formule (VII) 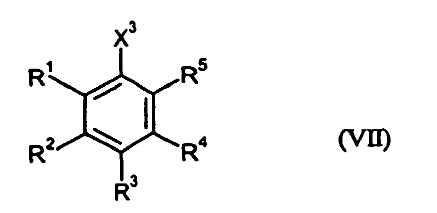 dans laquelle
R¹, R², R³, R⁴ et R⁵ ont les significations indiquées dans la revendication 1,
X³ représente le brome, l'iode ou un groupe trifluorométhylsulfonyloxy,
en présence d'un catalyseur, éventuellement en présence d'un liant acide et éventuellement en présence d'un agent de dilution.

14. Agent destiné à combattre des microorganismes non souhaités, **caractérisé par** une teneur en au moins un thiazolylcarboxanilide de la formule (I) selon la revendication 1 en plus de diluants et/ou de tensioactifs.

15. Utilisation de thiazolylcarboxanilides de la formule (I) selon la revendication 1 pour combattre des microorganismes non souhaités.

16. Procédé pour combattre des microorganismes non souhaités, **caractérisé en ce que** l'on applique des thiazolylcarboxanilides de la formule (I) selon la revendication 1 sur les microorganismes et/ou leur biotope.

17. Procédé pour la préparation d'agents destinés à combattre des microorganismes non souhaités, **caractérisé en ce que** l'on mélange des thiazolylcarboxanilides de la formule (I) selon la revendication 1 avec des diluants et/ou des tensioactifs.

18. Difluorométhylthiazolylcarboxyhalogénoanilides de la formule (IV) 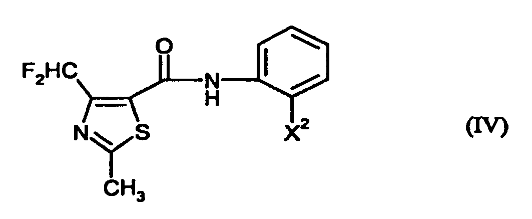 dans laquelle
X² représente le brome ou l'iode.

19. Procédé pour la préparation de difluorométhylthiazolylcarboxyhalogénoanilides de la formule (IV) selon la revendication 18, **caractérisé en ce que** l'on fait réagir
d) des halogénures d'acides difluorométhylthiazolylcarboxyliques de la formule (II) 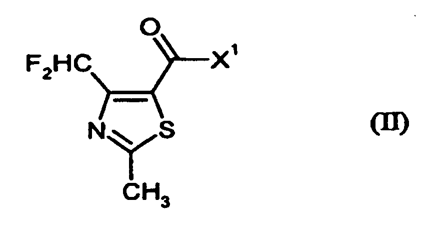 dans laquelle
X¹ représente un atome d'halogène,
avec de la 2-bromoaniline ou de la 2-iodoaniline.

20. Dérivés de difluorométhylthiazolylcarboxanilides-acides boriques de la formule (VI) 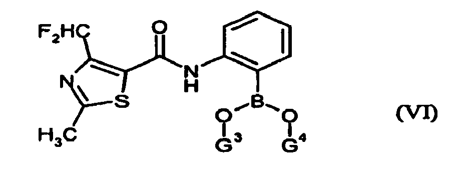 dans laquelle
G³ et G⁴ représentent chacun l'hydrogène ou ensemble un groupe tétraméthyléthylène.

21. Procédé pour la préparation de dérivés de difluorométhylthiazolylcarboxanilides-acides boriques de la formule (VI) selon la revendication 20, **caractérisé en ce que** l'on fait réagir
e) des halogénures d'acides difluorométhylthiazolylcarboxyliques de la formule (II) 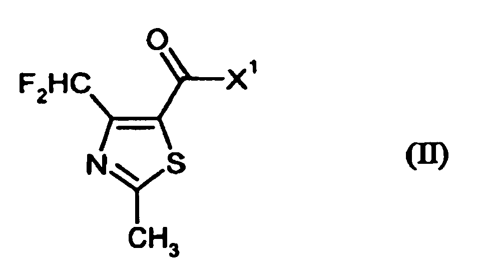 dans laquelle
X¹ représente un atome d'halogène,
avec des dérivés d'aniline-acides boriques de la formule (VIII) 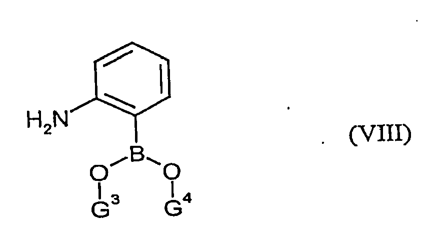 dans laquelle G³ et G⁴ ont les significations indiquées dans la revendication 20,
éventuellement en présence d'un liant acide et éventuellement en présence d'un agent de dilution.
